# EUROPEAN PATENT SPECIFICATION

(11) **EP 3 131 879 B1**
(45) Date of publication and mention of the grant of the patent: **13.09.2023**
(21) Application number: 15780679.5
(22) Date of filing: 17.04.2015
(51) Int. Cl.: H10K 85/60, C07D 209/86, C09K 11/06, C07F 15/00, C07D 209/88, C07D 401/04, C07D 401/10, C07D 401/14, C07D 405/14, C07D 409/14, C07D 487/04, C07D 491/04, C07D 495/04, H10K 50/12

(54) **MULTI-COMPONENT HOST MATERIAL AND AN ORGANIC ELECTROLUMINESCENCE DEVICE COMPRISING THE SAME**
MEHRKOMPONENTIGES HOST-MATERIAL UND ORGANISCHE ELEKTROLUMINESZENZVORRICHTUNG DAMIT
MATÉRIAU HÔTE À PLUSIEURS CONSTITUANTS ET DISPOSITIF ÉLECTROLUMINESCENT ORGANIQUE COMPRENANT LEDIT MATÉRIAU

(30) Priority: 18.04.2014 KR 20140046857; 11.07.2014 KR 20140087769
(43) Date of publication of application: 22.02.2017
(73) Proprietor: Rohm And Haas Electronic Materials Korea Ltd., Chungcheongnam-do 331-980 (KR)
(72) Inventor: AHN, Hee-Choon, Suwon-si Gyeonggi-do 443-400 (KR); KIM, Young-Kwang, Cheonan-si Chungcheongnam-do 331-980 (KR); LEE, Su-Hyun, Suwon-si Gyeonggi-do 440-200 (KR); JUN, Ji-Song, Suwon-si Gyeonggi-do 443-390 (KR); LEE, Seon-Woo, Chungcheongnam-do 331-980 (KR); KIM, Chi-Sik, Hwaseong-si Gyeonggi-do 445-752 (KR); PARK, Kyoung-Jin, Seongnam-si Gyeonggi-do 462-838 (KR); KIM, Nam-Kyun, Yongin-si Gyeonggi-do 448-527 (KR); CHOI, Kyung-Hoon, Hwaseong-si Gyeonggi-do 445-160 (KR); SHIM, Jae-Hoon, Chungcheongnam-do 331-980 (KR); CHO, Young-Jun, Seongnam-si Gyeonggi-do 463-400 (KR); LEE, Kyung-Joo, Seoul 121-773 (KR)
(74) Representative: Houghton, Mark Phillip
(86) International application number: PCT/KR2015/003890
(87) International publication number: WO 2015/160224

(56) References cited:
- EP-A1- 2 101 365
- EP-A1- 2 541 635
- WO-A1-2013/084885
- WO-A1-2013/146942
- WO-A1-2013/147205
- WO-A1-2013/151297
- WO-A1-2013/168688
- JP-A- 2009 029 726
- US-A1- 2013 214 258
- US-A1- 2013 234 119
- US-A1- 2013 234 119
- US-A1- 2014 299 865

## Description

### Technical Field

The present invention relates to a multi-component host material and an organic electroluminescence device comprising the same.

### Background Art

An electroluminescence device (EL device) is a self-light-emitting device which has advantages in that it provides a wider viewing angle, a greater contrast ratio, and a faster response time. An organic EL device was first developed by Eastman Kodak, by using small aromatic diamine molecules, and aluminum complexes as materials for forming a light-emitting layer [Appl. Phys. Lett. 51, 913, 1987].

An organic EL device (OLED) is a device changing electronic energy to light by applying electricity to an organic electroluminescent material, and generally has a structure comprising an anode, a cathode, and an organic layer between the anode and the cathode. The organic layer of an organic EL device may be comprised of, for example, a hole injection layer, a hole transport layer, an electron blocking layer, a light-emitting layer (which comprises host and dopant materials), an electron buffer layer, a hole blocking layer, an electron transport layer, and an electron injection layer, and the materials used for the organic layer are categorized by their functions in hole injection material, hole transport material, electron blocking material, light-emitting material, electron buffer material, hole blocking material, electron transport material, and electron injection material, for example. In the organic EL device, due to an application of a voltage, holes are injected from the anode to the light-emitting layer, electrons are injected from the cathode to the light-emitting layer, and excitons of high energies are formed by a recombination of the holes and the electrons. By this energy, luminescent organic compounds reach an excited state, and light emission occurs by emitting light from energy due to the excited state of the luminescent organic compounds returning to a ground state.

The most Important factor determining luminous efficiency in an organic EL device is the light-emitting material. A light-emitting material must have high quantum efficiency, high electron and hole mobility, and the formed light-emitting material layer must be uniform and stable. Light-emitting materials are categorized into blue, green, and red light-emitting materials dependent on the color of the light emission, additionally yellow or orange light-emitting materials. In addition, Light-emitting materials can also be categorized into host and dopant materials according to their functions. Recently, the development of an organic EL device providing high efficiency and long lifespan is an urgent issue. In particular, considering EL characteristic requirements for a middle or large-sized panel of OLED, materials showing better characteristics than conventional ones must be urgently developed. The host material which acts as a solvent in a solid state and transfers energy needs to have high purity and a molecular weight appropriate for vacuum deposition. Furthermore, the host material needs to have high glass transition temperature and high thermal degradation temperature to achieve thermal stability, high electro-chemical stability to achieve long lifespan, ease of forming amorphous thin film, good adhesion to materials of adjacent layers, and non-migration to other layers.

A light-emitting material can be used as a combination of a host and a dopant to improve color purity, luminous efficiency, and stability. Generally, an EL device having excellent characteristics has a structure comprising a light-emitting layer formed by doping a dopant to a host. Since host materials greatly influence the efficiency and lifespan of the EL device when using a dopant/host material system as a light emitting material, their selection is important.

International Publication Nos. WO 2013/168688 A1 and WO 2009/060757 A1, and Japanese Patent Appln. Laying-Open No. 2013-183036 A1, disclose organic electroluminescent devices using a biscarbazole derivative as a host material. However, the references fail to disclose an organic electroluminescent device using a multi-component host comprising a biscarbazole derivative and a carbazole derivative including a nitrogen-containing heteroaryl.

US 2013/234119 A1 and WO 2013/084885 A1 (US02014/0151647 A1) 2014/0151647) disclose an organic electroluminescence device employing a specific biscarbazole derivative having a cyano group as a first host and a compound having both a carbazole structure and a nitrogen-containing aromatic heteroring as a second host.

JP-A1-2009/029726 A discloses a compound having carbazolyl groups substituted with an aliphatic hydrocarbon group, a univalent aromatic hydrocarbon group or a univalent aromatic heterocyclic group, and with a halogen atom, a univalent organic residue or the like, for use as a blue light emitting material for an organic EL element.

US 2014/299865 A1 discloses an organic electroluminescence device includes an anode, a cathode and at least an emitting layer interposed between the anode and the cathode. The emitting layer contains a first host material, a second host material and a phosphorescent dopant material.

WO 2013/146942 A1 discloses a compound of general formula (1): 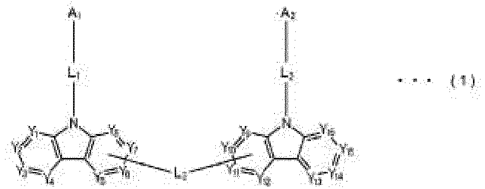 wherein Y1 through Y16 each independently represents CR or a nitrogen atom, and at least one of A1, A2, and R represents a substituted or unsubstituted condensed polycyclic aromatic hydrocarbon group. This organic electroluminescence element is provided with an organic thin film layer between a positive electrode and a negative electrode, and the organic thin film layer includes the aforementioned compound.

### Disclosure of the Invention

### Problems to be Solved

The objective of the present invention is to provide an organic electroluminescent device having high efficiency and long lifespan.

### Solution to Problems

The present inventors found that the above objective can be achieved by an organic electroluminescent device according to the claims comprising at least one light-emitting layer between an anode and a cathode, wherein the light-emitting layer comprises a host and a phosphorescent dopant, the host consists of multi-component host compounds, at least a first host compound of the multi-component host compounds is represented by the following formula 1, and a second host compound is represented by the following formula 2: 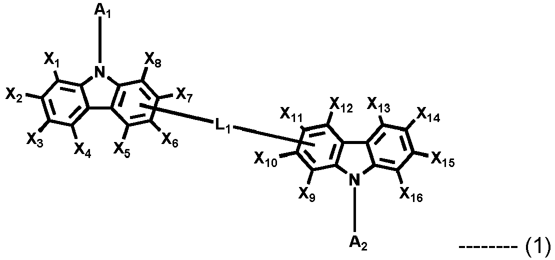 wherein
A₁ and A₂ each independently represent an unsubstituted (C6-C30)aryl selected from an unsubstituted phenyl, an unsubstituted naphthyl, or an unsubstituted biphenyl group;
L₁ represents a substituted or unsubstituted (C6-C30)arylene represented by any of the following formulae (7) to (11) or (13) to (19); 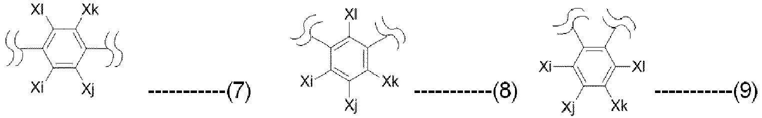 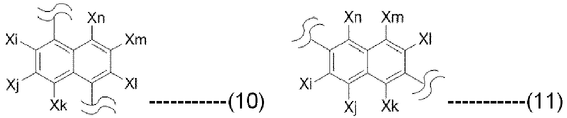 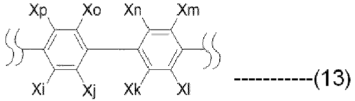 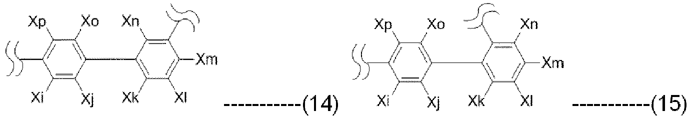 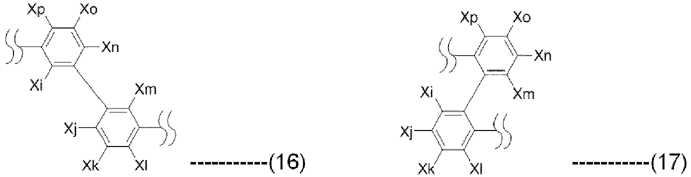 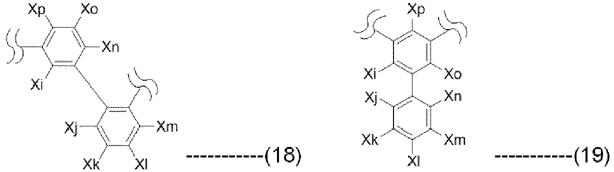
Xi to Xp each independently represent hydrogen, deuterium, a halogen, a substituted or unsubstituted (C1-C30)alkyl, a substituted or unsubstituted (C2-C30)alkenyl, a substituted or unsubstituted (C2-C30)alkynyl, a substituted or unsubstituted (C3-C30)cycloalkyl, a substituted or unsubstituted (C6-C60)aryl, a substituted or unsubstituted (3- to 30-membered)heteroaryl, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, or a substituted or unsubstituted mono- or di- (C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen and sulfur;
X₁ to X₁₆ each independently represent hydrogen, deuterium, a halogen, a substituted or unsubstituted (C1-C30)alkyl, a substituted or unsubstituted (C2-C30)alkenyl, a substituted or unsubstituted (C2-C30)alkynyl, a substituted or unsubstituted (C3-C30)cycloalkyl, a substituted or unsubstituted (C6-C60)aryl selected from the group consisting of phenyl, biphenyl, terphenyl, naphthyl, binaphthyl, phenylnaphthyl and naphthylphenyl, a substituted or unsubstituted (3- to 30-membered)heteroaryl, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, or a substituted or unsubstituted mono- or di- (C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen and sulfur;
the substituents on the substituted group of L1, X1 to X16 are deuterium, a halogen, a unsubstituted (CI-C30)alkyl group, unsubstituted (C6-30)aryl group, unsubstituted (3- to 30-membered)heteroaryl group, a tri(C6-C30)arylsilyl, a tri(Cl- C30)alkylsilyl, a di(Cl-C30)alkyl(C6-C30)arylsilyl, a (CI-C30)alkyldi(C6-C30)arylsilyl, an amino, a mono- or di(Cl-C30)alkylamino, a mono- or di(C6-C30)arylamino, a (Cl- C30)alkyl(C6-C30)arylamino, a (C6-C30)aryl(CI-C30)alkyl, or a (CI-C30)alkyl(C6- C30)aryl; 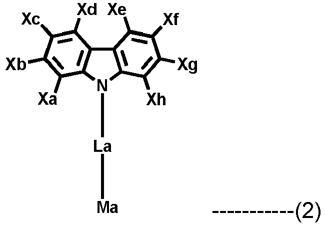 wherein
Ma represents a substituted or unsubstituted nitrogen-containing (5- to 11-membered)heteroaryl;
La represents a single bond, or a substituted or unsubstituted (C6-C30)arylene;
Xa to Xh each independently represent hydrogen, deuterium, a halogen, a substituted or unsubstituted (C1-C30)alkyl, a substituted or unsubstituted (C2-C30)alkenyl, a substituted or unsubstituted (C2-C30)alkynyl, a substituted or unsubstituted (C3-C30)cycloalkyl, a substituted or unsubstituted (C6-C60)aryl, a substituted or unsubstituted (3- to 30-membered)heteroaryl, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, or a substituted or unsubstituted mono- or di- (C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen and sulfur; and
the heteroaryl contains at least one hetero atom selected from B, N, O, S, P(=O), Si, and P; and
the substituents on the substituted group of Ma, La, Xa to Xh are deuterium, a halogen, a unsubstituted (C1-C30)alkyl group, unsubstituted (C6-30)aryl group, unsubstituted (3- to 30-membered)heteroaryl group, a tri(C6-C30)arylsilyl, a tri(C1-C30)alkylsilyl, a di(C1-C30)alkyl(C6-C30)arylsilyl, a (C1-C30)alkyldi(C6-C30)arylsilyl, an amino, a mono- or di(CI-C30)alkylamino, a mono- or di(C6-C30)arylamino, a (C1-C30)alkyl(C6-C30)arylamino, a (C6-C30)aryl(C1-C30)alkyl, or a (C1-C30)alkyl(C6- C30)aryl.

### Effects of the invention

According to the present invention, an organic electroluminescent device having high efficiency and long lifespan is provided, and it is possible to manufacture a display device or a lighting device using the organic electroluminescent device.

### Embodiments of the invention

Hereinafter, the present invention will be described in detail. However, the following description is intended to explain the invention, and is not meant in any way to restrict the scope of the invention.

Hereinafter, the organic electroluminescent device comprising the organic electroluminescent compounds of formulae 1 and 2 will be described in detail.

The compound represented by formula 1 can be represented by formula 3, 4, 5, or 6: 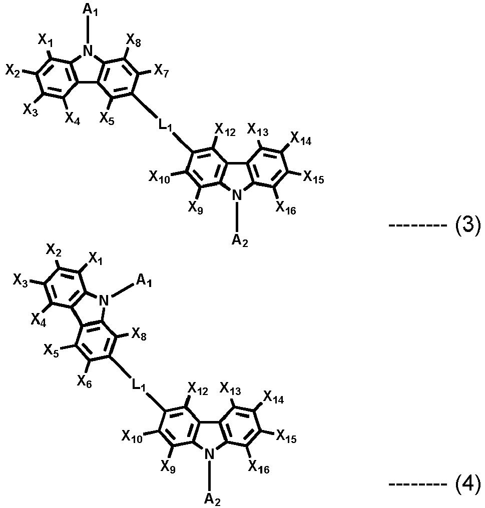 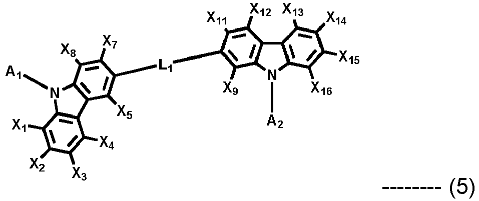 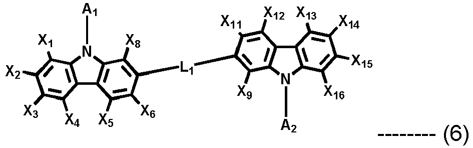 wherein
A₁, A₂, L₁, and X₁ to X₁₆ are as defined in formula 1.

In formula 1 above, A₁ and A₂ each independently represent an unsubstituted (C6-C30)aryl selected from an unsubstituted phenyl, an unsubstituted naphthyl, or an unsubstituted biphenyl group.

In formula 1 above, X₁ to X₁₆ each independently represent hydrogen, deuterium, a halogen, a substituted or unsubstituted (C1-C30)alkyl, a substituted or unsubstituted (C2-C30)alkenyl, a substituted or unsubstituted (C2-C30)alkynyl, a substituted or unsubstituted (C3-C30)cycloalkyl, a substituted or unsubstituted (C6-C60)aryl selected from the group consisting of phenyl, biphenyl, terphenyl, naphthyl, binaphthyl, phenylnaphthyl and naphthylphenyl, a substituted or unsubstituted (3- to 30-membered)heteroaryl, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, or a substituted or unsubstituted mono- or di- (C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen and sulfur.

In formula 1 above, L₁ represents a substituted or unsubstituted (C6-C30)arylene represented by any of the following formulae (7) to (11) or (13) to (19): 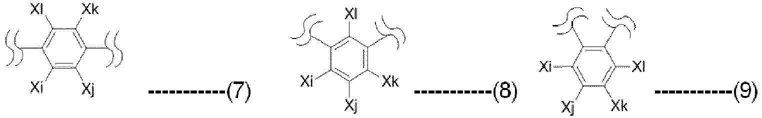 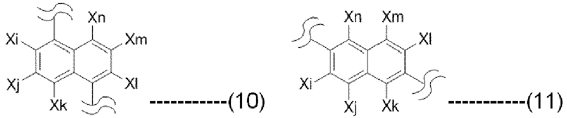 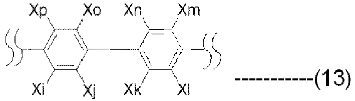 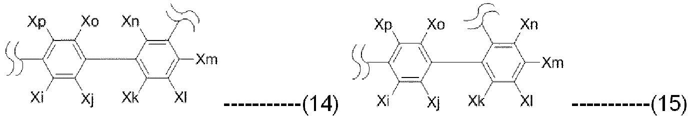 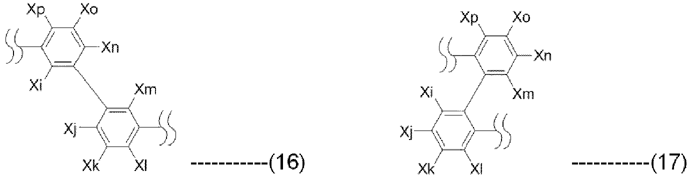 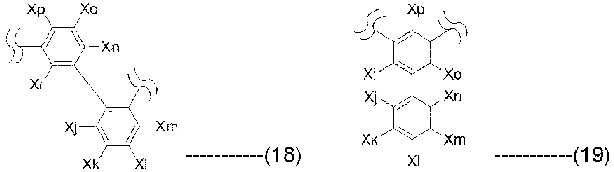 wherein
Xi to Xp each independently represent hydrogen, deuterium, a halogen, a substituted or unsubstituted (C1-C30)alkyl, a substituted or unsubstituted (C2-C30)alkenyl, a substituted or unsubstituted (C2-C30)alkynyl, a substituted or unsubstituted (C3-C30)cycloalkyl, a substituted or unsubstituted (C6-C60)aryl, a substituted or unsubstituted (3- to 30-membered)heteroaryl, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, or a substituted or unsubstituted mono- or di- (C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen and sulfur.

Preferably, Xi to Xp may each independently represent hydrogen, a halogen, a (C1-C10)alkyl, a (C3-C20)cycloalkyl, a (C6-C12)aryl, a (C1-C6)alkyldi(C6-C12)arylsilyl, or a tri(C6-C12)arylsilyl, and more preferably, each independently represent hydrogen, a (C1-C6)alkyl, or a tri(C6-C12)arylsilyl.

In formula 2 above, Ma represents a substituted or unsubstituted nitrogen-containing (5- to 11-membered)heteroaryl, preferably, represents a substituted or unsubstituted nitrogen-containing (6- to 10-membered)heteroaryl, and more preferably, represents a nitrogen-containing (6- to 10-membered)heteroaryl substituted with an unsubstituted (C6-C18)aryl, a (C6-C12)aryl substituted with a cyano, a (C6-C12)aryl substituted with a (C1-C6)alkyl, a (C6-C12)aryl substituted with a tri(C6-C12)arylsilyl, or a (6- to 15-membered)heteroaryl.

In addition, Ma may represent a monocyclic heteroaryl selected from the group consisting of pyrrolyl, imidazolyl, pyrazolyl, triazinyl, tetrazinyl, triazolyl, tetrazolyl, pyridyl, pyrazinyl, pyrimidinyl, and pyridazinyl, or a fused heteroaryl selected from the group consisting of benzoimidazolyl, isoindolyl, indolyl, indazolyl, benzothiadiazolyl, quinolyl, isoquinolyl, cinnolinyl, quinazolinyl, naphthyridinyl, and quinoxalinyl, and preferably may represent triazinyl, pyrimidinyl, pyridyl, quinolyl, isoquinolyl, quinazolinyl, naphthyridinyl, or quinoxalinyl.

In formula 2 above, La represents a single bond, or a substituted or unsubstituted (C6-C30)arylene, preferably, represents a single bond, or a substituted or unsubstituted (C6-C12)arylene, and more preferably, represents a single bond, or a (C6-C12)arylene unsubstituted or substituted with a tri(C6-C1 0)arylsilyl.

In addition, La can represent a single bond, or be represented by one of formulae (7) to (11) or (13) to 19 as above.

In formula 2 above, Xa to Xh each independently represent hydrogen, deuterium, a halogen, a substituted or unsubstituted (C1-C30)alkyl, a substituted or unsubstituted (C2-C30)alkenyl, a substituted or unsubstituted (C2-C30)alkynyl, a substituted or unsubstituted (C3-C30)cycloalkyl, a substituted or unsubstituted (C6-C60)aryl, a substituted or unsubstituted (3- to 30-membered)heteroaryl, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, or a substituted or unsubstituted mono- or di- (C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen and sulfur; and
the heteroaryl contains at least one hetero atom selected from B, N, O, S, P(=O), Si, and P;
the substituents on the substituted group of Ma, La, Xa to Xh are-deuterium, a halogen, a unsubstituted (C1-C30)alkyl group, unsubstituted (C6-30)aryl group, unsubstituted (3- to 30-membered)heteroaryl group, a tri(C6-C30)arylsilyl, a tri(C1-C30)alkylsilyl, a di(C1-C30)alkyl(C6-C30)arylsilyl, a (C1-C30)alkyldi(C6-C30)arylsilyl, an amino, a mono- or di(CI-C30)alkylamino, a mono- or di(C6-C30)arylamino, a (C1-C30)alkyl(C6-C30)arylamino, a (C6-C30)aryl(C1-C30)alkyl, or a (C1-C30)alkyl(C6-C30)aryl.

Herein, "(C1-C30)alkyl" is meant to be a linear or branched alkyl having 1 to 30 carbon atoms, in which the number of carbon atoms is preferably 1 to 20, more preferably 1 to 10, and includes methyl, ethyl, n-propyl, isopropyl, n-butyl, isobutyl, and tert-butyl; "(C2-C30)alkenyl" is meant to be a linear or branched alkenyl having 2 to 30 carbon atoms, in which the number of carbon atoms is preferably 2 to 20, more preferably 2 to 10, and includes vinyl, 1-propenyl, 2-propenyl, 1-butenyl, 2-butenyl, 3-butenyl, and 2-methylbut-2-enyl; "(C2-C30)alkynyl" is meant to be a linear or branched alkynyl having 2 to 30 carbon atoms, in which the number of carbon atoms is preferably 2 to 20, more preferably 2 to 10, and includes ethynyl, 1-propynyl, 2-propynyl, 1-butynyl, 2-butynyl, 3-butynyl, and 1-methylpent-2-ynyl; "(C3-C30)cycloalkyl" is a mono- or polycyclic hydrocarbon having 3 to 30 carbon atoms, in which the number of carbon atoms is preferably 3 to 20, more preferably 3 to 7, and includes cyclopropyl, cyclobutyl, cyclopentyl, and cyclohexyl; "(3- to 7-membered)heterocycloalkyl" is a cycloalkyl having 3 to 7 ring backbone atoms, preferably 5 to 7, including at least one heteroatom selected from B, N, O, S, P(=O), Si and P, preferably O, S and N, and includes tetrahydrofuran, pyrrolidine, thiolan, and tetrahydropyran; "(C6-C30)aryl(ene)" is a monocyclic or fused ring derived from an aromatic hydrocarbon having 6 to 30 carbon atoms, in which the number of carbon atoms is preferably 6 to 20, more preferably 6 to 15, and includes phenyl, biphenyl, terphenyl, naphthyl, binaphthyl, phenylnaphthyl, naphthylphenyl, fluorenyl, phenylfluorenyl, benzofluorenyl, dibenzofluorenyl, phenanthrenyl, phenylphenanthrenyl, anthracenyl, indenyl, triphenylenyl, pyrenyl, tetracenyl, perylenyl, chrysenyl, naphthacenyl, and fluoranthenyl; "(3- to 30-membered)heteroaryl" is an aryl having 3 to 30 ring backbone atoms, including at least one, preferably 1 to 4 heteroatoms selected from the group consisting of B, N, O, S, P(=O), Si and P; is a monocyclic ring, or a fused ring condensed with at least one benzene ring; may be partially saturated; may be one formed by linking at least one heteroaryl or aryl group to a heteroaryl group via a single bond(s); and includes a monocyclic ring-type heteroaryl including furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, and pyridazinyl, and a fused ring-type heteroaryl including benzofuranyl, benzothiophenyl, isobenzofuranyl, dibenzofuranyl, dibenzothiophenyl, benzoimidazolyl, benzothiazolyl, benzoisothiazolyl, benzoisoxazolyl, benzoxazolyl, isoindolyl, indolyl, benzoindolyl, indazolyl, benzothiadiazolyl, quinolyl, isoquinolyl, cinnolinyl, quinazolinyl, quinoxalinyl, carbazolyl, phenoxazinyl, phenanthridinyl, and benzodioxolyl; "nitrogen-containing (5- to 30-membered)heteroaryl" is an aryl having 5 to 30 ring backbone atoms, preferably 5 to 20, and more preferably 5 to 15, including at least one heteroatom, N; is a monocyclic ring, or a fused ring condensed with at least one benzene ring; may be partially saturated; may be one formed by linking at least one heteroaryl or aryl group to a heteroaryl group via a single bond(s); and includes a monocyclic ring-type heteroaryl including pyrrolyl, imidazolyl, pyrazolyl, triazinyl, tetrazinyl, triazolyl, tetrazolyl, pyridyl, pyrazinyl, pyrimidinyl, and pyridazinyl, and a fused ring-type heteroaryl including benzoimidazolyl, isoindolyl, indolyl, indazolyl, benzothiadiazolyl, quinolyl, isoquinolyl, cinnolinyl, quinazolinyl, quinoxalinyl, carbazolyl, and phenanthridinyl. Further, "halogen" includes F, Cl, Br, and I.

Herein, "substituted" in the expression "substituted or unsubstituted" means that a hydrogen atom in a certain functional group is replaced with another atom or group, i.e. a substituent. The substituents of the substituted alkyl, the substituted alkenyl, the substituted alkynyl, the substituted cycloalkyl, the substituted aryl(ene), the substituted heteroaryl, the substituted trialkylsilyl, the substituted triarylsilyl, the substituted dialkylarylsilyl, the substituted alkyldiarylsilyl, the substituted mono- or di- arylamino, and the substituted nitrogen-containing heteroaryl in L₁, X₁ to X₁₆, Ma, La, and Xa to Xh in formulae 1 and 2 each independently are at least one selected from the group consisting of deuterium, a halogen, a unsubstituted (C1-C30)alkyl group, unsubstituted (C6-30)aryl group, unsubstituted (3- to 30-membered)heteroaryl group, a tri(C6-C30)arylsilyl, a tri(C1-C30)alkylsilyl, a di(C1-C30)alkyl(C6-C30)arylsilyl, a (C1-C30)alkyldi(C6-C30)arylsilyl, an amino, a mono- or di(C1-C30)alkylamino, a mono- or di(C6-C30)arylamino, a (C1-C30)alkyl(C6-C30)arylamino, a (C6-C30)aryl(C1-C30)alkyl, or a (C1-C30)alkyl(C6-C30)aryl;, and preferably are at least one selected from the group consisting of a (C1-C6)alkyl, a (5- to 15-membered)heteroaryl, a (C6-C18)aryl unsubstituted or substituted with a a tri(C6-C12)arylsilyl, a tri(C6-C12)arylsilyl, and a (C1-C6)alkyl(C6-C12)aryl.

In formula 1, a triarylsilyl as X₁ to X₁₆ is preferably a triphenylsilyl.

The first host compound represented by formula 1 includes the compounds listed in accompanying claim 6, but is not limited thereto.

The second host compound represented by formula 2 includes the compounds listed in accompanying claim 7, but is not limited thereto.

The organic electroluminescent device according to the present invention comprises an anode; a cathode; and at least one organic layer between the anode and the cathode. The organic layer comprises a light-emitting layer, and the light-emitting layer comprises a host and a phosphorescent dopant. The host consists of multi-component host compounds, at least a first host compound of the multi-component host compounds is represented by formula 1, and a second host compound is represented by formula 2.

The light-emitting layer is a layer from which light is emitted, and can be a single layer or a multi layer of which two or more layers are stacked. In the light-emitting layer, it is preferable that the doping concentration of the dopant compound based on the host compound is less than 20 wt%.

The organic layer comprises a light-emitting layer, and may further comprise at least one layer selected from the group consisting of a hole injection layer, a hole transport layer, an electron transport layer, an electron injection layer, an interlayer, a hole blocking layer, and an electron blocking layer.

According to the organic electroluminescent device of the present invention, the weight ratio of the first host material to the second host material is in the range of 1:99 to 99:1.

The dopant is preferably at least one phosphorescent dopant. The dopant materials applied to the organic electroluminescent device according to the present invention are not limited, but may be preferably selected from metallated complex compounds of iridium, osmium, copper and platinum, more preferably selected from ortho-metallated complex compounds of iridium, osmium, copper and platinum, and even more preferably ortho-metallated iridium complex compounds.

The phosphorescent dopant is preferably selected from compounds represented by the following formulae 101 to 103. 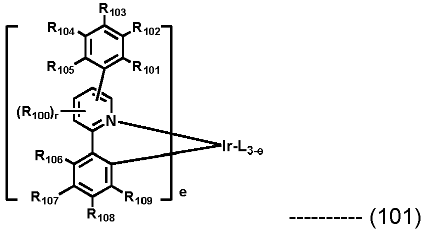 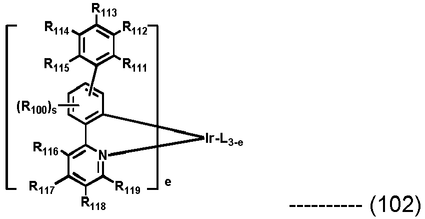 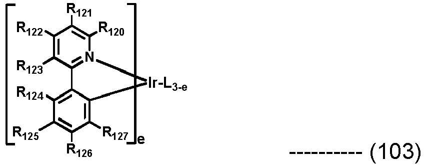 wherein L is selected from the following structures: 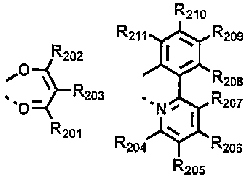
R₁₀₀ represents hydrogen, a substituted or unsubstituted (C1-C30)alkyl, or a substituted or unsubstituted (C3-C30)cycloalkyl;
R₁₀₁ to R₁₀₉, and R₁₁₁ to R₁₂₃ each independently represent hydrogen, deuterium, a halogen, a (C1-C30)alkyl unsubstituted or substituted with deuterium or a halogen(s), a cyano, a substituted or unsubstituted (C1-C30)alkoxy, a substituted or unsubstituted (C6-C30)aryl, or a substituted or unsubstituted (C3-C30)cycloalkyl; adjacent substituents of R₁₀₆ to R₁₀₉ may be linked to each other to form a substituted or unsubstituted fused ring, e.g., fluorene unsubstituted or substituted with alkyl, dibenzothiophene unsubstituted or substituted with alkyl, or dibenzofuran unsubstituted or substituted with alkyl; and adjacent substituents of R₁₂₀ to R₁₂₃ may be linked to each other to form a substituted or unsubstituted fused ring, e.g., quinoline unsubstituted or substituted with halogen, alkyl, or aryl;
R₁₂₄ to R₁₂₇ each independently represent hydrogen, deuterium, a halogen, a substituted or unsubstituted (C1-C30)alkyl, or a substituted or unsubstituted (C6-C30)aryl; and adjacent substituents of R₁₂₄ to R₁₂₇ may be linked to each other to form a substituted or unsubstituted fused ring, e.g., fluorene unsubstituted or substituted with alkyl, dibenzothiophene unsubstituted or substituted with alkyl, or dibenzofuran unsubstituted or substituted with alkyl;
R₂₀₁ to R₂₁₁ each independently represent hydrogen, deuterium, a halogen, a (C1-C30)alkyl unsubstituted or substituted with deuterium or a halogen(s), a substituted or unsubstituted (C3-C30)cycloalkyl, or a substituted or unsubstituted (C6-C30)aryl; and adjacent substituents of R₂₀₈ to R₂₁₁ may be linked to each other to form a substituted or unsubstituted fused ring, e.g., fluorene unsubstituted or substituted with alkyl, dibenzothiophene unsubstituted or substituted with alkyl, or dibenzofuran unsubstituted or substituted with alkyl;
r and s each independently represent an integer of 1 to 3; where r or s is an integer of 2 or more, each of R₁₀₀ may be the same or different; and e represents an integer of 1 to 3.

Specifically, the phosphorescent dopant materials include the following:

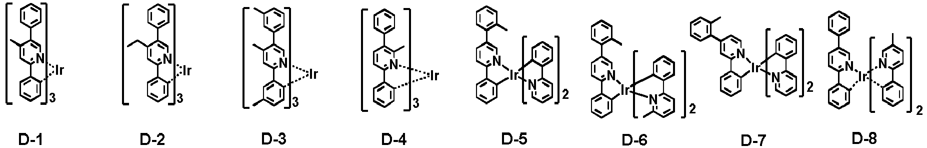

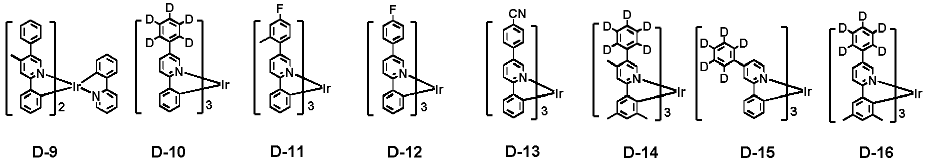

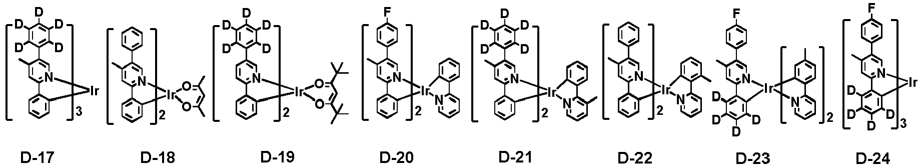

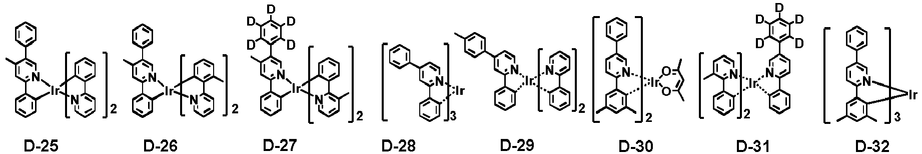

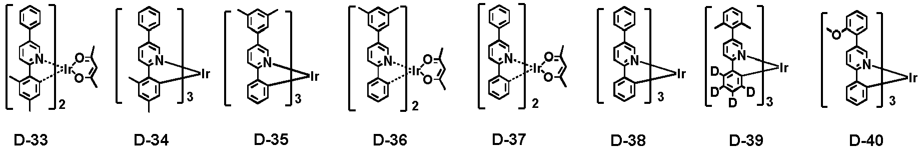

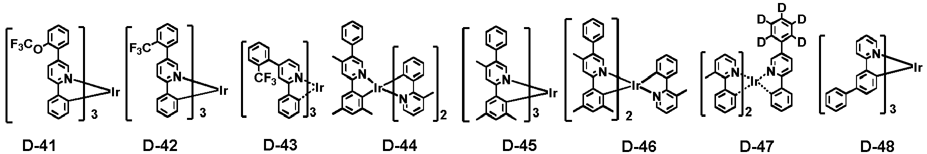

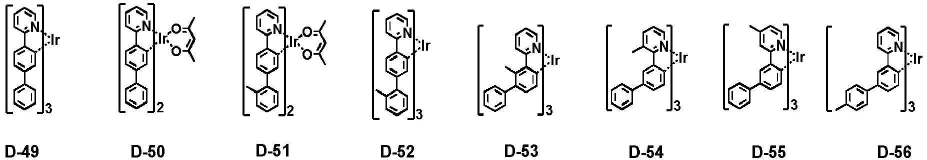

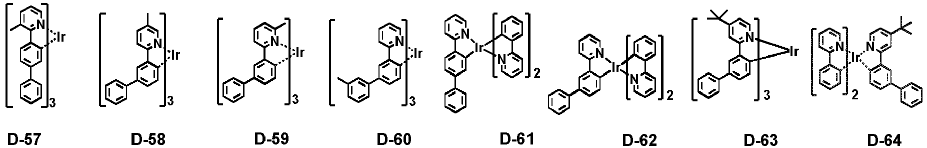

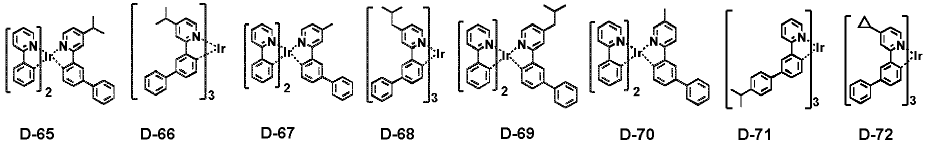

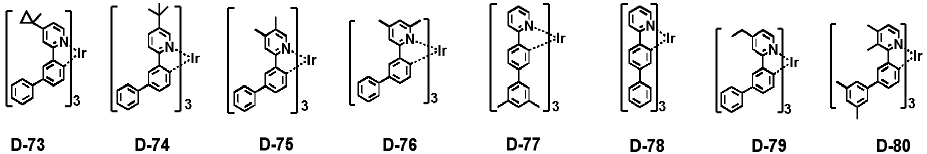

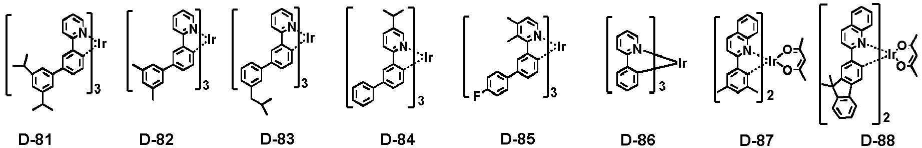

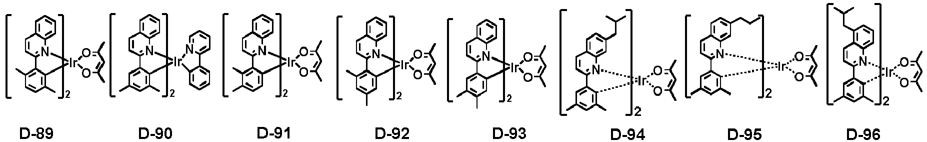

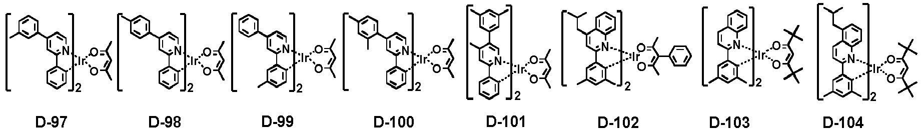

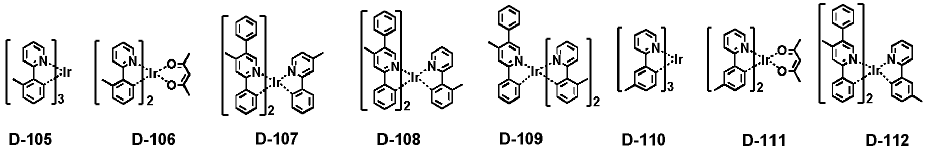

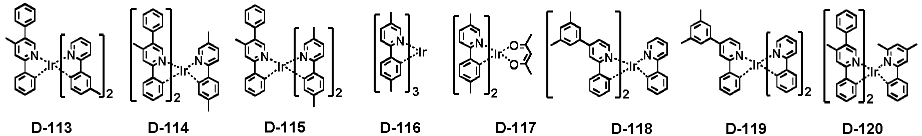

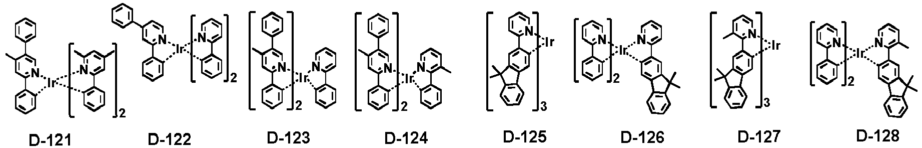

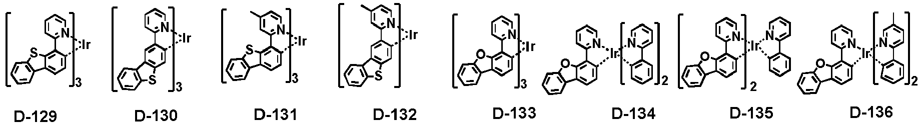

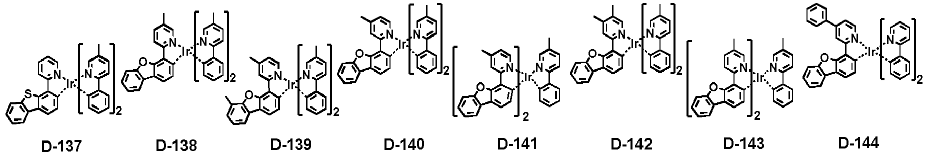

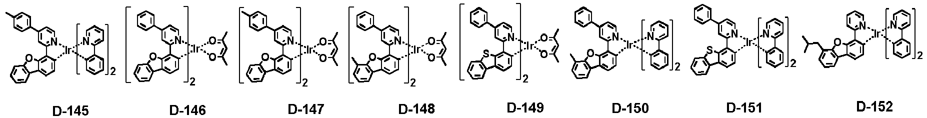

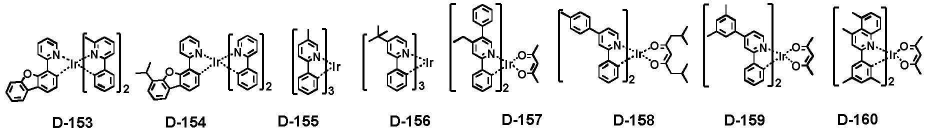

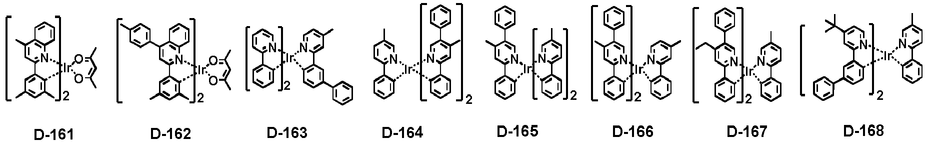

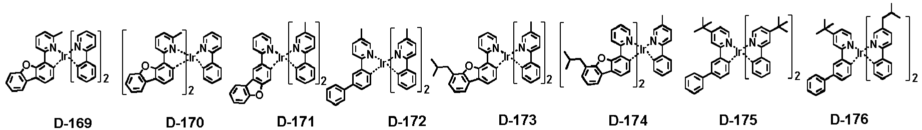

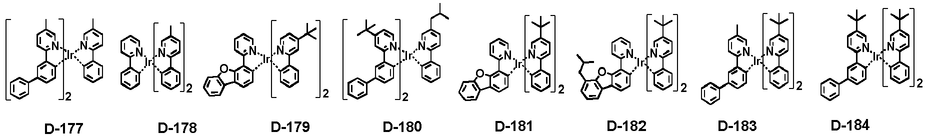

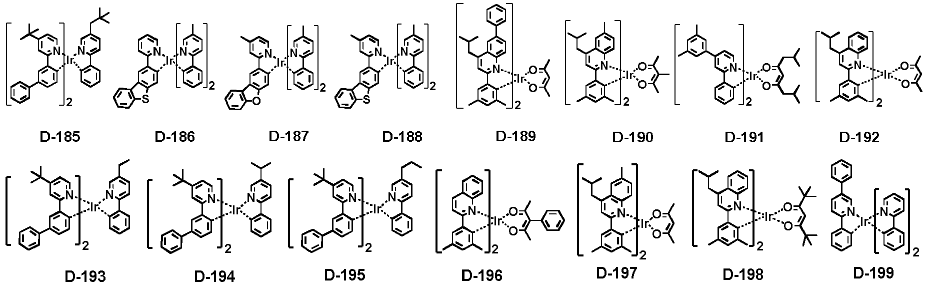

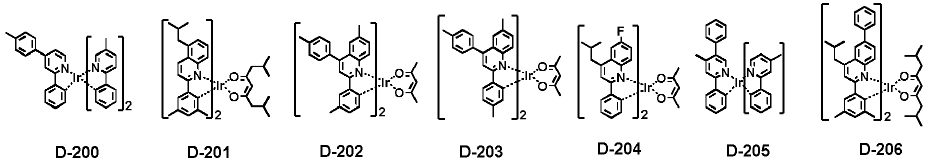

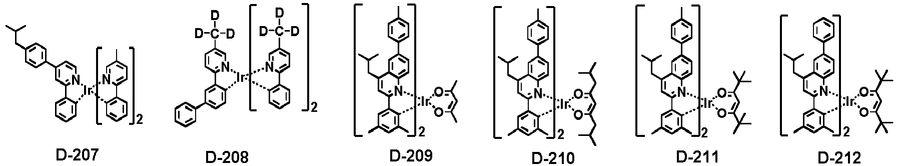

The organic electroluminescent device according to the present invention may further comprise at least one compound selected from the group consisting of arylamine-based compounds and styrylarylamine-based compounds in the organic layer.

In addition, in the organic electroluminescent device according to the present invention, the organic layer may further comprise at least one metal selected from the group consisting of metals of Group 1, metals of Group 2, transition metals of the 4^{th} period, transition metals of the 5^{th} period, lanthanides and organic metals of d-transition elements of the Periodic Table, or at least one complex compound comprising said metal.

According to the present invention, at least one layer (hereinafter, "a surface layer") is preferably placed on an inner surface(s) of one or both electrode(s); selected from a chalcogenide layer, a metal halide layer and a metal oxide layer. Specifically, a chalcogenide (including oxides) layer of silicon or aluminum is preferably placed on an anode surface of an electroluminescent medium layer, and a metal halide layer or a metal oxide layer is preferably placed on a cathode surface of an electroluminescent medium layer. Such a surface layer provides operation stability for the organic electroluminescent device. Preferably, said chalcogenide includes SiO_{X}(1≤5X≤2), AlO_{X}(1≤X≤1.5), SiON, and/or SiAlON; said metal halide includes LiF, MgF₂, CaF₂, and/or a rare earth metal fluoride; and said metal oxide includes Cs₂O, Li₂O, MgO, SrO, BaO, and/or CaO.

Between the anode and the light-emitting layer, a layer selected from a hole injection layer, a hole transport layer, or an electron blocking layer, or formed by a combination thereof can be used. Multi layers can be used for the hole injection layer in order to lower the hole injection barrier (or hole injection voltage) from the anode to the hole transport layer or the electron blocking layer. Two compounds can be simultaneously used in each layer. The hole transport layer and the electron blocking layer can also be formed of multi layers.

Between the light-emitting layer and the cathode, a layer selected from an electron buffer layer, a hole blocking layer, an electron transport layer, or an electron injection layer, or formed by a combination thereof can be used. Multi layers can be used for the electron buffer layer in order to control the injection of the electrons and enhance the interfacial characteristics between the light-emitting layer and the electron injection layer. Two compounds can be simultaneously used in each layer. The hole blocking layer and the electron transport layer can also be formed of multi layers, and each layer can comprise two or more compounds.

In the organic electroluminescent device according to the present invention, a mixed region of an electron transport compound and a reductive dopant, or a mixed region of a hole transport compound and an oxidative dopant is preferably placed on at least one surface of a pair of electrodes. In this case, the electron transport compound is reduced to an anion, and thus it becomes easier to inject and transport electrons from the mixed region to an electroluminescent medium. Further, the hole transport compound is oxidized to a cation, and thus it becomes easier to inject and transport holes from the mixed region to the electroluminescent medium. Preferably, the oxidative dopant includes various Lewis acids and acceptor compounds; and the reductive dopant includes alkali metals, alkali metal compounds, alkaline earth metals, rare-earth metals, and mixtures thereof. A reductive dopant layer may be employed as a charge generating layer to prepare an electroluminescent device having two or more electroluminescent layers and emitting white light.

In order to form each layer of the organic electroluminescent device of the present invention, dry film-forming methods such as vacuum evaporation, sputtering, plasma and ion plating methods, or wet film-forming methods such as ink jet printing, nozzle printing, slot coating, spin coating, dip coating, and flow coating methods can be used. The first and second host compounds of the present invention may be co-evaporated or mixture-evaporated.

When using a wet film-forming method, a thin film can be formed by dissolving or diffusing materials forming each layer into any suitable solvent such as ethanol, chloroform, tetrahydrofuran, or dioxane. The solvent can be any solvent where the materials forming each layer can be dissolved or diffused, and where there are no problems in film-formation capability.

Herein, a co-evaporation indicates a process for two or more materials to be deposited as a mixture, by introducing each of the two or more materials into respective crucible cells, and applying an electric current to the cells for each of the materials to be evaporated. Herein, a mixture-evaporation indicates a process for two or more materials to be deposited as a mixture, by mixing the two or more materials in one crucible cell before the deposition, and applying an electric current to the cell for the mixture to be evaporated.

By using the organic electroluminescent device of the present invention, a display system or a lighting system can be produced.

Hereinafter, the luminescent properties of the device comprising the host compound of the present invention will be explained in detail with reference to the following examples.

### Device Examples 1-1 to 1-6: Preparation of an OLED device by co-evaporating the first host compound and the second host compound of the present invention

An OLED device was produced using the organic electroluminescent compound according to the present invention. A transparent electrode indium tin oxide (ITO) thin film (10 Ω/sq) on a glass substrate for an organic light-emitting diode (OLED) device (Geomatec) was subjected to an ultrasonic washing with trichloroethylene, acetone, ethanol, and distilled water, sequentially, and then was stored in isopropanol. The ITO substrate was then mounted on a substrate holder of a vacuum vapor depositing apparatus. N⁴,N^{4'}-diphenyl-N⁴,N⁴-bis(9-phenyl-9H-carbazol-3-yl)-[1,1'-biphenyl]-4,4'-diamine (compound HI-1) was introduced into a cell of said vacuum vapor depositing apparatus, and then the pressure in the chamber of said apparatus was controlled to 10⁻⁶ torr. Thereafter, an electric current was applied to the cell to evaporate the above introduced material, thereby forming a first hole injection layer having a thickness of 80 nm on the ITO substrate. Next, 1,4,5,8,9,12-hexaazatriphenylene-hexacarbonitrile (compound HI-2) was introduced into another cell of said vacuum vapor depositing apparatus, and was evaporated by applying an electric current to the cell, thereby forming a second hole injection layer having a thickness of 5 nm on the first hole injection layer. N-([1,1'-biphenyl]-4-yl)-9,9-dimethyl-N-(4-(9-phenyl-9H-carbazol-3-yl)phenyl)-9H-fluorene-2-amine (compound HT-1) was then introduced into another cell of said vacuum vapor depositing apparatus, and was evaporated by applying an electric current to the cell, thereby forming a first hole transport layer having a thickness of 10 nm on the second hole injection layer. Afterwards, N,N-di([1,1'-biphenyl]-4-yl)-4'-(9H-carbazol-9-yl)-[1,1'-biphenyl]-4-amine (compound HT-2) was introduced into another cell of said vacuum vapor depositing apparatus, and was evaporated by applying an electric current to the cell, thereby forming a second hole transport layer having a thickness of 60 nm on the first hole transport layer. As a host material, a first host compound and a second host compound were introduced into two cells of the vacuum vapor depositing apparatus, respectively. A dopant compound D-96 was introduced into another cell. The two host materials were evaporated at 1:1 rate, while the dopant was evaporated at a different rate from the host materials, so that the dopant was deposited in a doping amount of 3 wt% based on the total amount of the host and dopant to form a light-emitting layer having a thickness of 40 nm on the hole transport layer. 2,4-bis(9,9-dimethyl-9H-fluoren-2-yl)-6-(naphthalen-2-yl)-1,3,5-triazine (compound ET-1) and lithium quinolate (compound El-1) were then introduced into two cells of the vacuum vapor depositing apparatus, respectively, and evaporated at 1:1 rate to form an electron transport layer having a thickness of 30 nm on the light-emitting layer. After depositing lithium quinolate (compound El-1) as an electron injection layer having a thickness of 2 nm on the electron transport layer, an Al cathode having a thickness of 80 nm was deposited by another vacuum vapor deposition apparatus. Thus, an OLED device was produced.

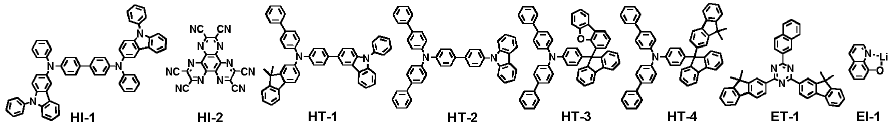

### Comparative Examples 1-1 to 1-3: Preparation of an OLED device using only the second host compound as a host

An OLED device was produced in the same manner as in Device Examples 1-1 to 1-6, except for using only the second host compound as a host of the light-emitting layer.

The driving voltage at 1,000 nit, luminous efficiency, CIE color coordinate, and the time taken for the luminance at 5,000 nit to be reduced from 100% to 80% at a constant current of the OLEDs produced as above were measured.

Table 1 below shows the luminous characteristics of the organic electroluminescent devices produced as in the examples above.

**Table 1**

| Device No. | HTL | Host | Dopant | Voltage [V] | Efficiency [cd/A] | Color Coordinate (x,y) | Lifespan [hr] |
|---|---|---|---|---|---|---|---|
| Example 1-1 | HT-1 / HT-2 | H1-1 : H2-2 | D-96 | 4.4 | 27.5 | 0.664, 0.335 | 1,280 |
| Example 1-2 | HT-1 / HT-2 | H1-7 : H2-29 | D-96 | 3.9 | 27.7 | 0.665, 0.332 | 550 |
| Reference example 1-3 | HT-1 / HT-2 | H1-19 : H2-29 | D-96 | 4.1 | 24.6 | 0.664, 0.332 | 470 |
| Reference Example 1-4 | HT-1 / HT-2 | H1-36 : H2-154 | D-96 | 4.6 | 27.8 | 0.663, 0.335 | 1,530 |
| Reference Example 1-5 | HT-1 / HT-2 | H1-36 : H2-29 | D-96 | 3.7 | 28.2 | 0.666, 0.331 | 780 |
| Reference Example 1-6 | HT-1 / HT-2 | H1-36 : H2-155 | D-96 | 4.6 | 26.9 | 0.664, 0.335 | 970 |
| Comp. Ex. 1-1 | HT-1 / HT-2 | H2-2 | D-96 | 4.1 | 28.2 | 0.662, 0.337 | 300 |
| Comp. Ex. 1-2 | HT-1 / HT-2 | H2-154 | D-96 | 4.5 | 27.1 | 0.662, 0.337 | 420 |
| Comp. Ex. 1-3 | HT-1 / HT-2 | H2-29 | D-96 | 3.6 | 27.5 | 0.668, 0.331 | 310 |

### Device Examples 2-1 to 2-7: Preparation of an OLED device by co-evaporating the first host compound and the second host compound of the present invention

An OLED device was produced in the same manner as in Device Examples 1-1 to 1-6, except for forming the second hole injection layer of 3 nm; forming the first hole transport layer of 40 nm; not forming the second hole transport layer; doping compound D-25 as the dopant of the light-emitting layer in a doping amount of 15 wt% based on the total amount of the host and dopant; forming the electron transport layer of 35 nm by evaporating 2,4-bis(9,9-dimethyl-9H-fluoren-2-yl)-6-(naphthalen-2-yl)-1,3,5-triazine and lithium quinolate at a rate of 4:6; and using other combinations for the first host compound and the second host compound used in the host of the light-emitting layer.

### Device Examples 2-8 to 2-9: Preparation of an OLED device by co-evaporating the first host compound and the second host compound of the present invention

An OLED device was produced in the same manner as in Device Examples 1-1 to 1-6, except for forming the second hole injection layer of 3 nm; forming the first hole transport layer of 40 nm; not forming the second hole transport layer; doping compound D-1 as the dopant of the light-emitting layer in a doping amount of 15 wt% based on the total amount of the host and dopant; forming the electron transport layer of 35 nm by evaporating 2,4-bis(9,9-dimethyl-9H-fluoren-2-yl)-6-(naphthalen-2-yl)-1,3,5-triazine and lithium quinolate at a rate of 4:6; and using other combinations for the first host compound and the second host compound used in the host of the light-emitting layer.

### Device Example 2-10: Preparation of an OLED device by co-evaporating the first host compound and the second host compound of the present invention

An OLED device was produced in the same manner as in Device Examples 1-1 to 1-6, except for forming the second hole injection layer of 3 nm; forming the first hole transport layer of 40 nm; not forming the second hole transport layer; doping compound D-136 as the dopant of the light-emitting layer in a doping amount of 15 wt% based on the total amount of the host and dopant; forming the electron transport layer of 35 nm by evaporating 2,4-bis(9,9-dimethyl-9H-fluoren-2-yl)-6-(naphthalen-2-yl)-1,3,5-triazine and lithium quinolate at a rate of 4:6; and using other combinations for the first host compound and the second host compound used in the host of the light-emitting layer.

### Device Examples 3-1 to 3-3: Preparation of an OLED device by co-evaporating the first host compound and the second host compound of the present invention

An OLED device was produced in the same manner as in Device Examples 2-1 to 2-7, except for forming the first hole injection layer of 10 nm; forming the second hole transport layer of 30 nm using compound HT-3; using compound D-136 as the dopant of the light-emitting layer; and using other combinations for the first host compound and the second host compound used in the host of the light-emitting layer.

### Device Example 3-4: Preparation of an OLED device by co-evaporating the first host compound and the second host compound of the present invention

An OLED device was produced in the same manner as in Device Examples 2-1 to 2-7, except for forming the first hole injection layer of 10 nm; forming the second hole transport layer of 30 nm using compound HT-3; using compound D-168 as the dopant of the light-emitting layer; and using other combinations for the first host compound and the second host compound used in the host of the light-emitting layer.

### Comparative Examples 2-1 to 2-3: Preparation of an OLED device using only the first host compound as a host

An OLED device was produced in the same manner as in Device Examples 2-1 to 2-7, except for using only the first host compound as a host of the light-emitting layer.

### Comparative Examples 3-1 to 3-3: Preparation of an OLED device using only the second host compound as a host

An OLED device was produced in the same manner as in Device Examples 2-1 to 2-7, except for using only the second host compound as a host of the light-emitting layer.

### Comparative Examples 3-4 to 3-6: Preparation of an OLED device using only the second host compound as a host

An OLED device was produced in the same manner as in Device Examples 2-8 to 2-9, except for using only the second host compound as a host of the light-emitting layer.

### Comparative Examples 4-1 to 4-3: Preparation of an OLED device using only the second host compound as a host

An OLED device was produced in the same manner as in Device Examples 3-1 to 3-3, except for using only the second host compound as a host of the light-emitting layer.

The driving voltage at 1,000 nit, luminous efficiency, CIE color coordinate, and the time taken for the luminance at 15,000 nit to be reduced from 100% to 80% at a constant current of the OLEDs produced as above were measured.

Table 2 below shows the luminous characteristics of the organic electroluminescent devices produced as in the examples above.

**[Table 2]**

| Device No. | HTL | Host | Dopant | Voltage [V] | Efficiency [cd/A] | Color Coordinate (x,y) | Lifespan [hr] |
|---|---|---|---|---|---|---|---|
| Example 2-1 | HT-1 | H1-1 : H2-25 | D-25 | 3.2 | 49.8 | 0.301, 0.658 | 350 |
| Example 2-2 | HT-1 | H1-1 : H2-31 | D-25 | 3 | 57.1 | 0.308, 0.655 | 380 |
| Example 2-3 | HT-1 | H1-1 : H2-48 | D-25 | 2.9 | 56.8 | 0.305, 0.656 | 400 |
| Example 2-4 | HT-1 | H1-1 : H2-101 | D-25 | 3 | 55.5 | 0.303, 0.657 | 230 |
| Example 2-5 | HT-1 | H1-1 : H2-34 | D-25 | 3.1 | 58.1 | 0.306, 0.655 | 440 |
| Reference Example 2-6 | HT-1 | H1-4: H2-31 | D-25 | 3 | 53.3 | 0.304, 0.656 | 120 |
| Example 2-7 | HT-1 | H1-37 : H2-31 | D-25 | 3 | 53.7 | 0.306, 0.655 | 300 |
| Example 2-8 | HT-1 | H1-1 : H2-31 | D-1 | 2.9 | 53 | 0.321, 0.656 | 560 |
| Example 2-9 | HT-1 | H1-1 : H2-48 | D-1 | 2.8 | 55.3 | 0.319, 0.657 | 550 |
| Example 2-10 | HT-1 | H1-113: H2-31 | D-136 | 2.8 | 59.4 | 0.331, 0.655 | 600 |
| Example 3-1 | HT-1 / HT-3 | H1-1 : H2-48 | D-136 | 3.1 | 67.5 | 0.326, 0.658 | 590 |
| Example 3-2 | HT-1 / HT-3 | H1-1 : H2-273 | D-136 | 3.1 | 66.2 | 0.328, 0.657 | 700 |
| Example 3-3 | HT-1 / HT-3 | H1-113: H2-125 | D-136 | 3.1 | 65.8 | 0.329, 0.657 | 700 |
| Example 3-4 | HT-1 / HT-3 | H1-1 : H2-273 | D-168 | 3.0 | 57.2 | 0.288, 0.665 | 450 |
| Comp. Ex. 2-1 | HT-1 | H1-1 | D-25 | 6.8 | 3.1 | 0.301, 0.653 | × |
| Comp. Ex. 2-2 | HT-1 | H1-4 | D-25 | 7.2 | 3.6 | 0.295, 0.658 | × |
| Comp. Ex. 2-3 | HT-1 | H 1-37 | D-25 | 7.0 | 3.0 | 0.302, 0.653 | × |
| Comp. Ex. 3-1 | HT-1 | H2-31 | D-25 | 2.9 | 42.8 | 0.314, 0.652 | 100 |
| Comp. Ex. 3-2 | HT-1 | H2-101 | D-25 | 2.8 | 50.3 | 0.315, 0.651 | 60 |
| Comp. Ex. 3-3 | HT-1 | H2-34 | D-25 | 2.7 | 49.2 | 0.312, 0.652 | 100 |
| Comp. Ex. 3-4 | HT-1 | H2-31 | D-1 | 2.9 | 33.5 | 0.323, 0.653 | 390 |
| Comp. Ex. 3-5 | HT-1 | H2-48 | D-1 | 2.6 | 41.2 | 0.325, 0.653 | 380 |
| Comp. Ex. 3-6 | HT-1 | H2-87 | D-1 | 2.8 | 37.9 | 0.323, 0.653 | 420 |
| Comp. Ex. 4-1 | HT-1 / HT-3 | H2-48 | D-136 | 2.6 | 51.9 | 0.334, 0.652 | 490 |
| Comp. Ex. 4-2 | HT-1 / HT-3 | H2-125 | D-136 | 3.0 | 64.9 | 0.337, 0.649 | 360 |
| Comp. Ex. 4-3 | HT-1 / HT-3 | H2-273 | D-136 | 3.3 | 68.2 | 0.332, 0.654 | 440 |

### Device Example 4-1: Preparation of an OLED device by co-evaporating the first host compound and the second host compound of the present invention

An OLED device was produced in the same manner as in Device Examples 1-1 to 1-6, except for using compound HT-4 for the second hole transport layer, and using the compounds as listed in Table 3 below for the first host compound and the second host compound used in the host of the light-emitting layer.

### Comparative Example 5-1: Preparation of an OLED device using only the second host compound as a host

An OLED device was produced in the same manner as in Device Example 4-1, except for using only the second host compound of Table 3 as a host of the light-emitting layer.

The driving voltage at 1,000 nit, luminous efficiency, CIE color coordinate, and the time taken for the luminance at 5,000 nit to be reduced from 100% to 90% at a constant current of the OLEDs produced as above were measured.

Table 3 below shows the luminous characteristics of the organic electroluminescent devices produced as in the examples above.

**[Table 3]**

| Device No. | HTL | Host | Dopant | Voltage [V] | Efficiency [cd/A] | Color Coordinate (x,y) | Lifespan [hr] |
|---|---|---|---|---|---|---|---|
| Example 4-1 | HT-1 / HT-4 | H1-7 : H2-41 | D-96 | 3.4 | 30.7 | 0.665, 0.333 | 400 |
| Comp. Ex. 5-1 | HT-1 / HT-4 | H2-41 | D-96 | 3.1 | 28.3 | 0.668, 0.331 | 300 |

The organic electroluminescent device of the present invention comprises a light-emitting layer comprising a host and a phosphorus dopant, and the host consists of a specific combination of multi-component host compounds. The device of the present invention provides superior lifespan characteristics to conventional devices.

## Claims

1. An organic electroluminescent device comprising at least one light-emitting layer between an anode and a cathode, wherein the light-emitting layer comprises a host and a phosphorescent dopant, the host consists of multi-component host compounds, at least a first host compound of the multi-component host compounds is represented by the following formula 1, and a second host compound is represented by the following formula 2. 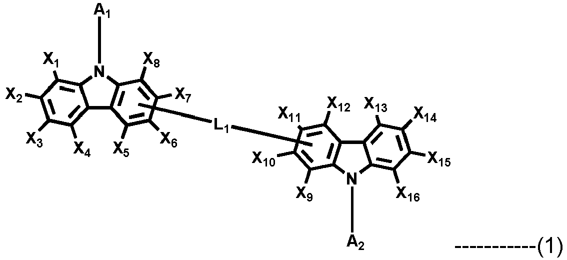 wherein
A₁ and A₂ each independently represent an unsubstituted (C6-C30)aryl selected from an unsubstituted phenyl, an unsubstituted naphthyl, or an unsubstituted biphenyl group;
L₁ represents a substituted or unsubstituted (C6-C30)arylene represented by any of the following formulae (7) to (11) or (13) to (19); 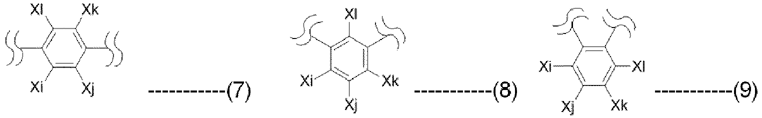 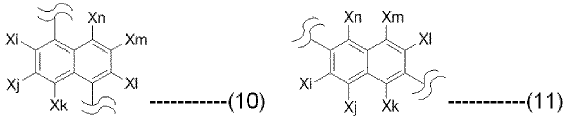 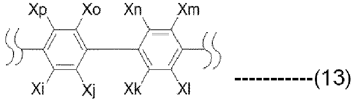 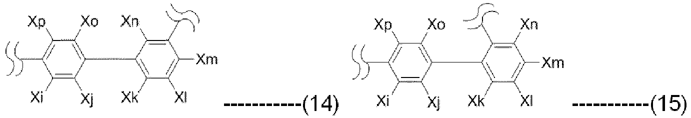 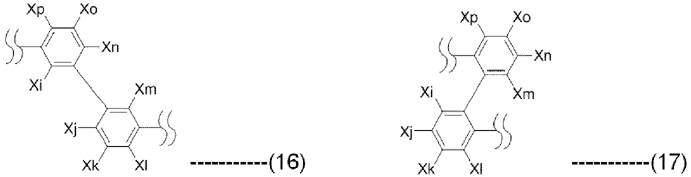 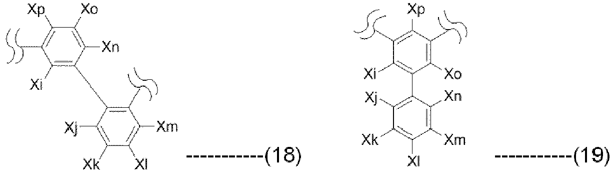
Xi to Xp each independently represent hydrogen, deuterium, a halogen, a substituted or unsubstituted (C1-C30)alkyl, a substituted or unsubstituted (C2-C30)alkenyl, a substituted or unsubstituted (C2-C30)alkynyl, a substituted or unsubstituted (C3-C30)cycloalkyl, a substituted or unsubstituted (C6-C60)aryl, a substituted or unsubstituted (3- to 30-membered)heteroaryl, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, or a substituted or unsubstituted mono- or di- (C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen and sulfur;
X₁ to X₁₆ each independently represent hydrogen, deuterium, a halogen, a substituted or unsubstituted (C1-C30)alkyl, a substituted or unsubstituted (C2-C30)alkenyl, a substituted or unsubstituted (C2-C30)alkynyl, a substituted or unsubstituted (C3-C30)cycloalkyl, a substituted or unsubstituted (C6-C60)aryl selected from the group consisting of phenyl, biphenyl, terphenyl, naphthyl, binaphthyl, phenylnaphthyl and naphthylphenyl, a substituted or unsubstituted (3- to 30-membered) heteroaryl, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, or a substituted or unsubstituted mono- or di- (C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen and sulfur;
the substituents on the substituted group of L1, X1 to X16 are deuterium, a halogen, a unsubstituted (C1-C30)alkyl group, unsubstituted (C6-C30)aryl group, unsubstituted (3- to 30-membered)heteroaryl group, a tri(C6-C30)arylsilyl, a tri(C1- C30)alkylsilyl, a di(C1-C30)alkyl(C6-C30)arylsilyl, a (C1-C30)alkyldi(C6-C30)arylsilyl, an amino, a mono- or di(C1-C30)alkylamino, a mono- or di(C6-C30)arylamino, a (C1- C30)alkyl(C6-C30)arylamino, a (C6-C30)aryl(C1-C30)alkyl, or a (C1-C30)alkyl(C6- C30)aryl; 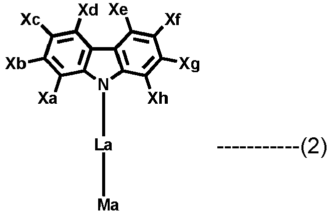 wherein
Ma represents a substituted or unsubstituted nitrogen-containing (5- to 11-membered) heteroaryl;
La represents a single bond, or a substituted or unsubstituted (C6-C30)arylene;
Xa to Xh each independently represent hydrogen, deuterium, a halogen, a substituted or unsubstituted (C1-C30)alkyl, a substituted or unsubstituted (C2-C30)alkenyl, a substituted or unsubstituted (C2-C30)alkynyl, a substituted or unsubstituted (C3-C30)cycloalkyl, a substituted or unsubstituted (C6-C60)aryl, a substituted or unsubstituted (3- to 30-membered)heteroaryl, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, or a substituted or unsubstituted mono- or di- (C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen and sulfur; and
the heteroaryl contains at least one hetero atom selected from B, N, O, S, P(=O), Si, and P; and
the substituents on the substituted group of Ma, La, Xa to Xh are deuterium, a halogen, a unsubstituted (C1-C30)alkyl group, unsubstituted (C6-C30)aryl group, unsubstituted (3- to 30-membered)heteroaryl group, a tri(C6-C30)arylsilyl, a tri(C1-C30)alkylsilyl, a di(C1-C30)alkyl(C6-C30)arylsilyl, a (C1-C30)alkyldi(C6-C30)arylsilyl, an amino, a mono- or di(C1-C30)alkylamino, a mono- or di(C6-C30)arylamino, a (C1-C30)alkyl(C6-C30)arylamino, a (C6-C30)aryl(C1-C30)alkyl, or a (C1-C30)alkyl(C6-C30)aryl.

2. The organic electroluminescent device according to claim 1, wherein in formula 2, Ma represents a monocyclic heteroaryl selected from the group consisting of pyrrolyl, imidazolyl, pyrazolyl, triazinyl, tetrazinyl, triazolyl, tetrazolyl, pyridyl, pyrazinyl, pyrimidinyl, and pyridazinyl, or a fused heteroaryl selected from the group consisting of benzoimidazolyl, isoindolyl, indolyl, indazolyl, benzothiadiazolyl, quinolyl, isoquinolyl, cinnolinyl, quinazolinyl, naphthyridinyl, and quinoxalinyl.

3. The organic electroluminescent device according to claim 1, wherein in formula 2, La is a single bond, or represented by one of the following formulae 7 to 19: 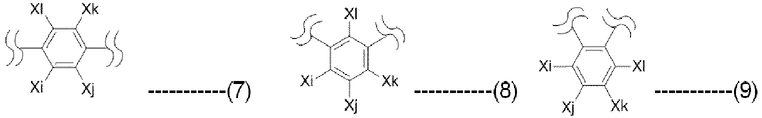 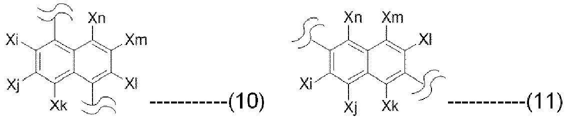 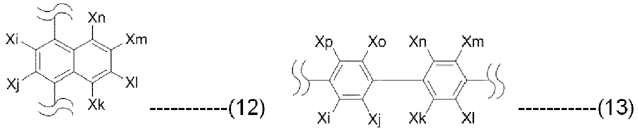 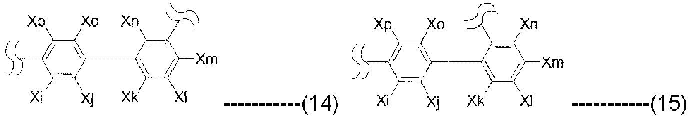 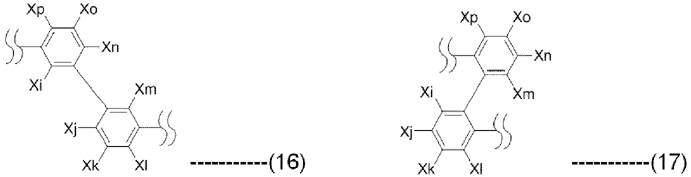 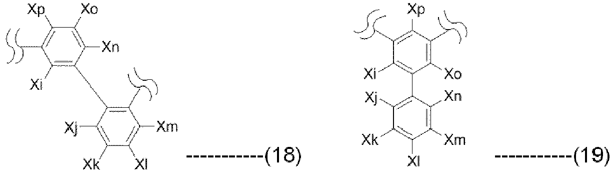 wherein
Xi to Xp each independently represent hydrogen, deuterium, a halogen, a substituted or unsubstituted (C1-C30)alkyl, a substituted or unsubstituted (C2-C30)alkenyl, a substituted or unsubstituted (C2-C30)alkynyl, a substituted or unsubstituted (C3-C30)cycloalkyl, a substituted or unsubstituted (C6-C60)aryl, a substituted or unsubstituted (3- to 30-membered)heteroaryl, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, or a substituted or unsubstituted mono- or di- (C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen and sulfur.

4. The organic electroluminescent device according to claim 1, wherein in formula 2, Xa to Xh each independently represent hydrogen; a (C6-C15)aryl unsubstituted or substituted with a tri(C6-C10)arylsilyl; a (10- to 20-membered)heteroaryl unsubstituted or substituted with a (C6-C12)aryl or a cyano(C6-C12)aryl; or an unsubstituted tri(C6-C10)arylsilyl; or are linked to an adjacent substituent(s) to form a substituted or unsubstituted benzene, a substituted or unsubstituted indole, a substituted or unsubstituted benzoindole, a substituted or unsubstituted indene, a substituted or unsubstituted benzofuran, or a substituted or unsubstituted benzothiophene.

5. The organic electroluminescent device according to claim 1, wherein in formula 1, a triarylsilyl as X₁ to X₁₆ is a triphenylsilyl.

6. The organic electroluminescent device according to claim 1, wherein the compound represented by formula 1 is selected from the group consisting of:

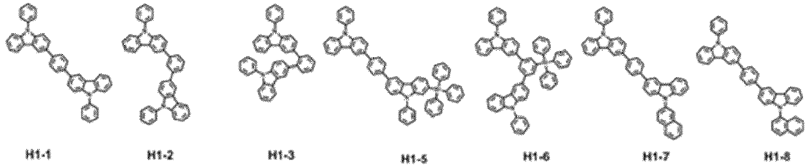

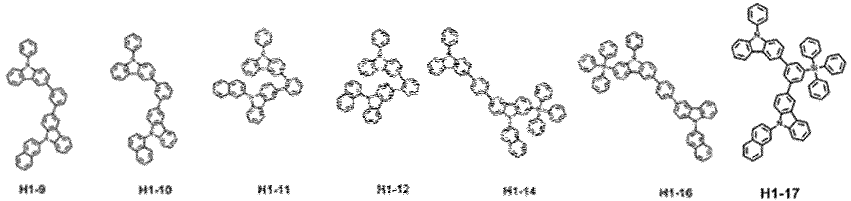

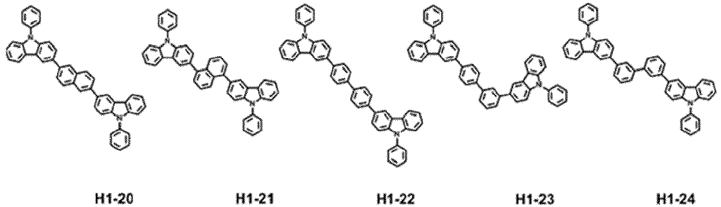

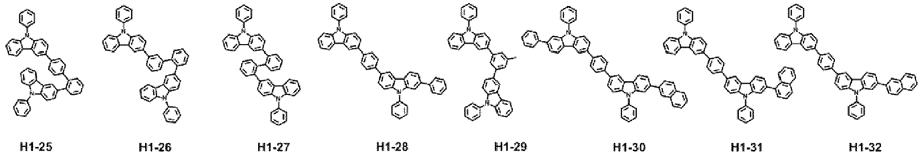

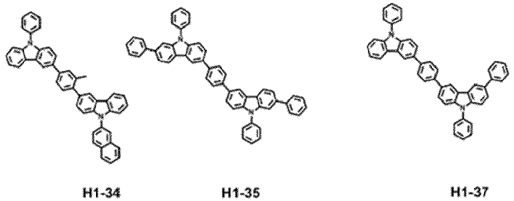

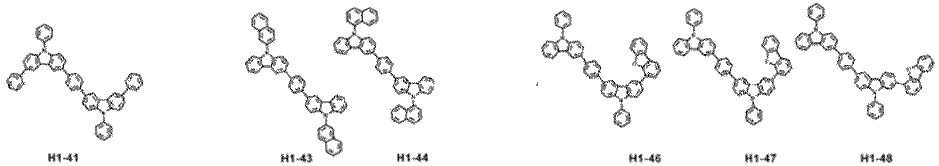

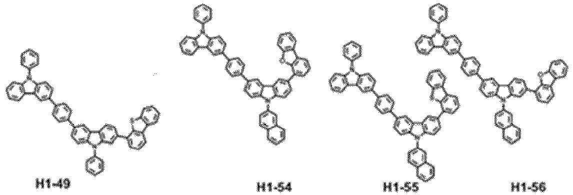

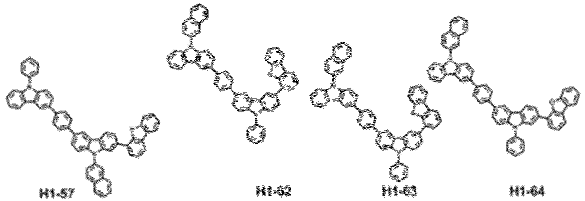

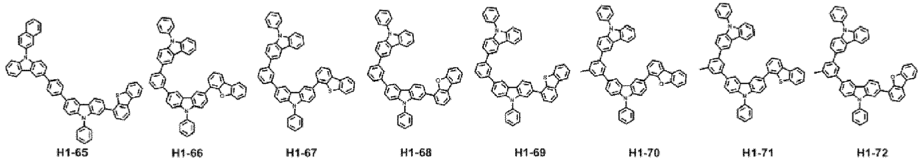

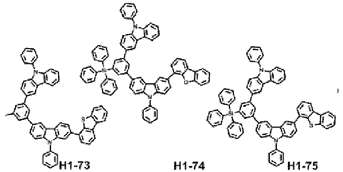

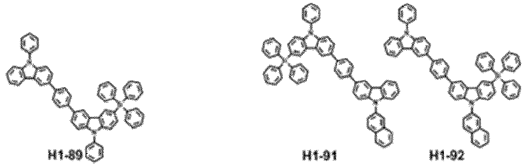

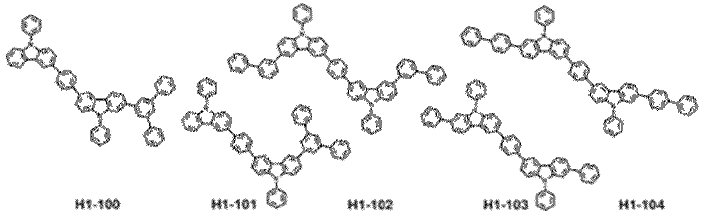

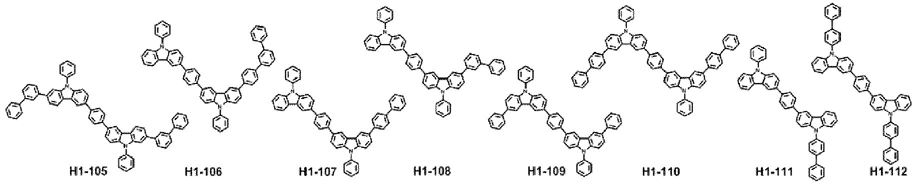

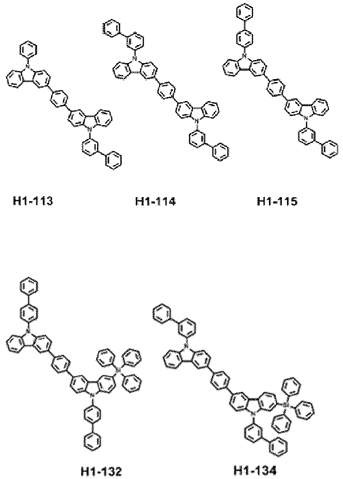

7. The organic electroluminescent device according to claim 1, wherein the compound represented by formula 2 is selected from the group consisting of:

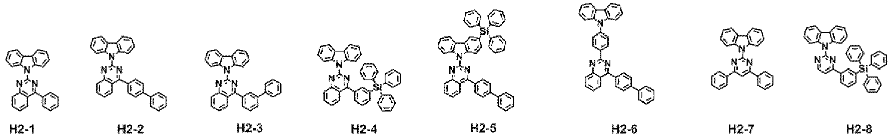

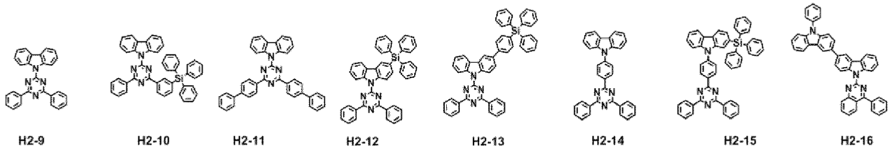

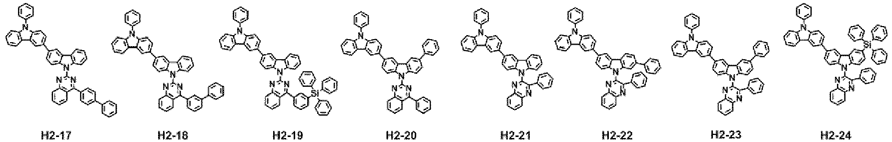

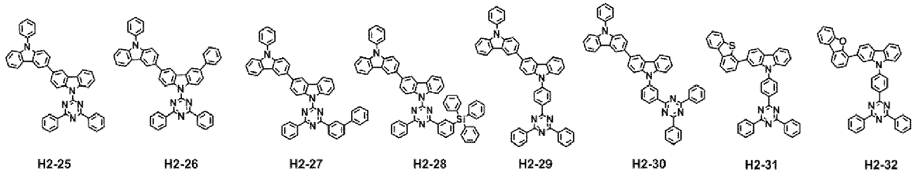

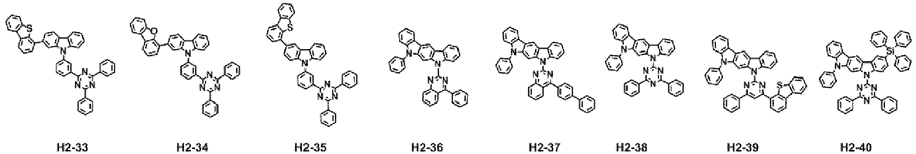

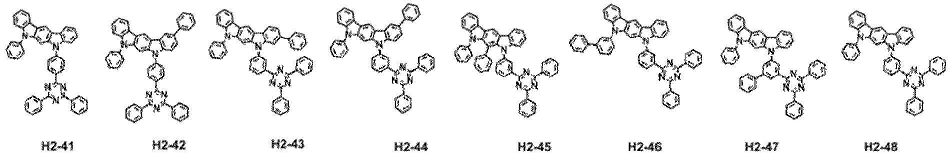

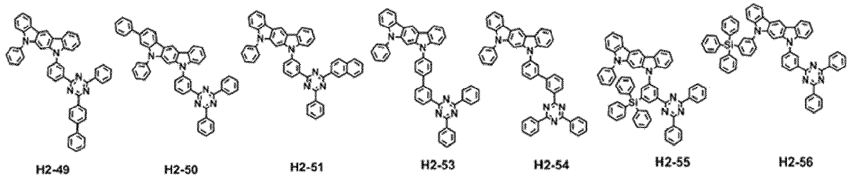

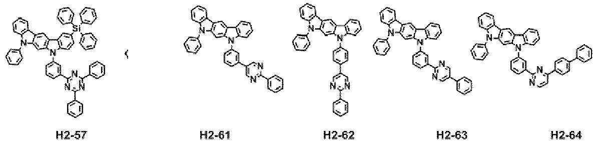

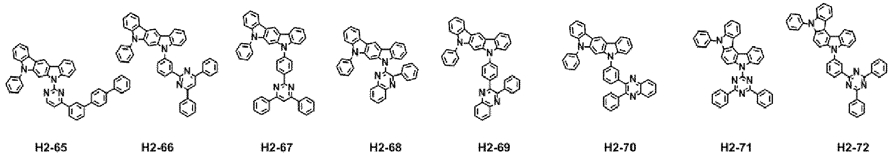

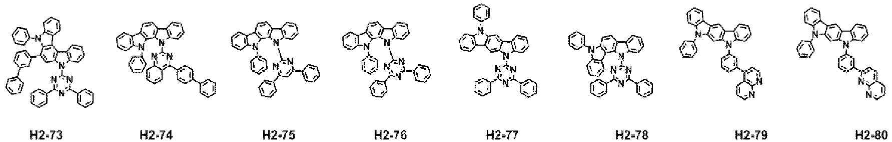

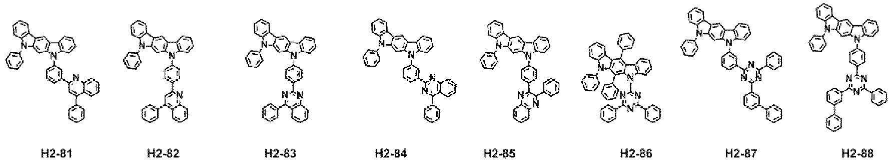

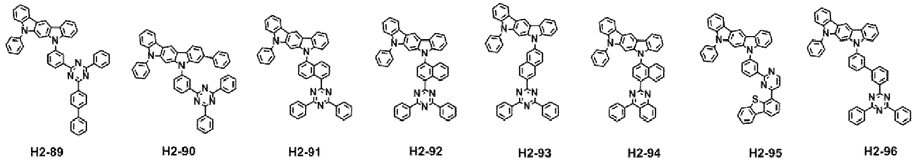

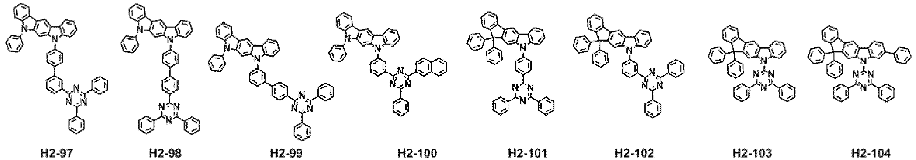

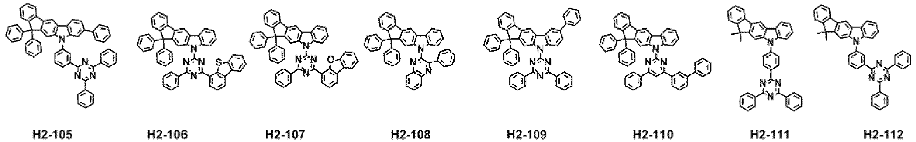

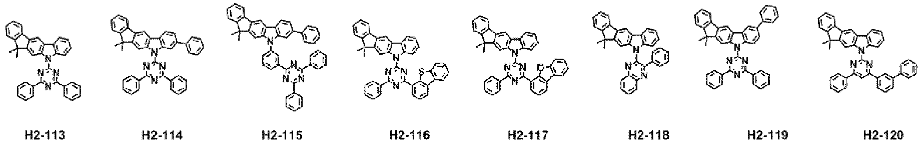

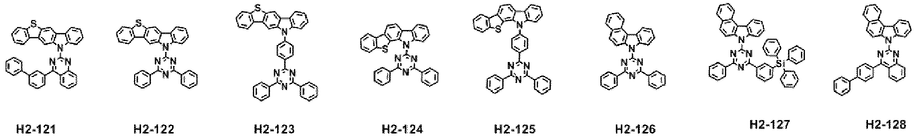

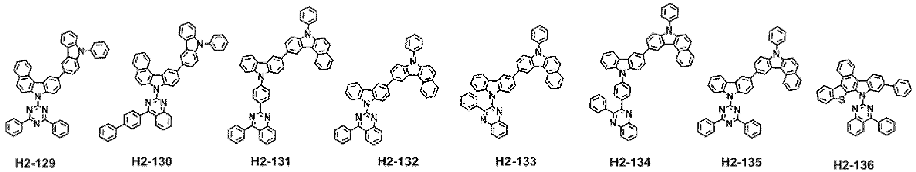

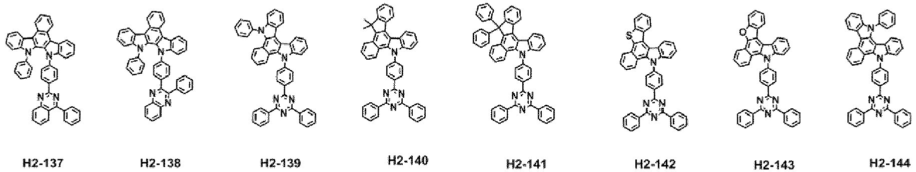

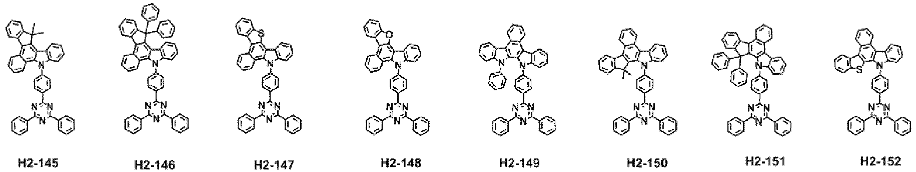

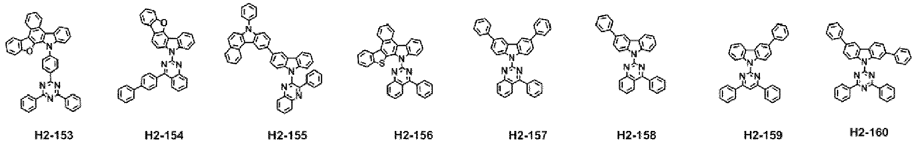

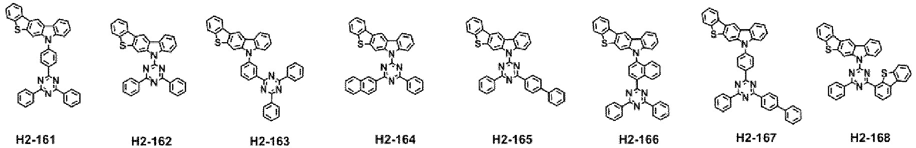

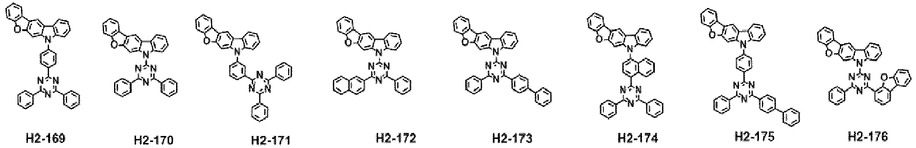

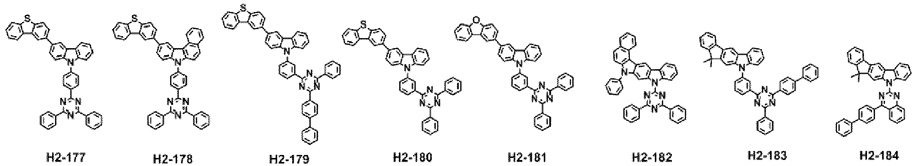

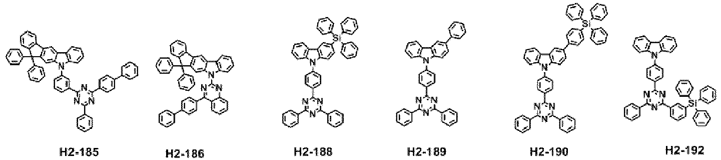

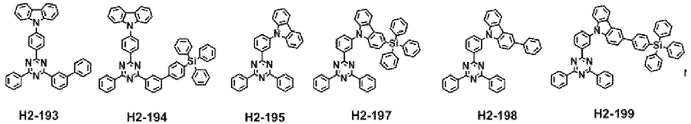

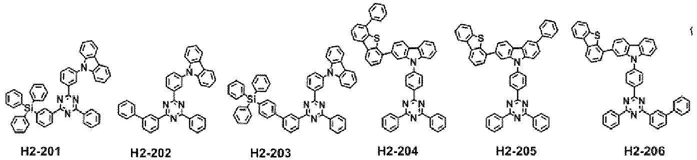

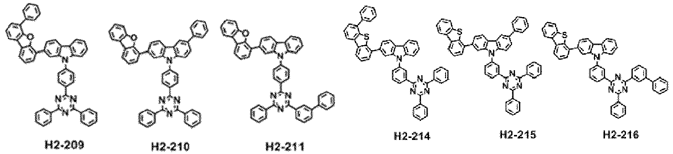

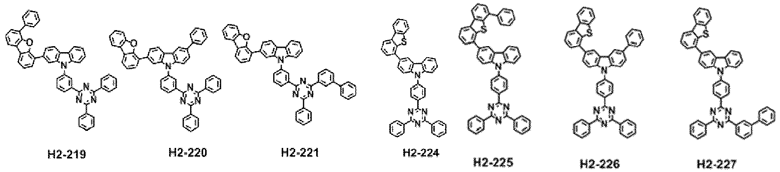

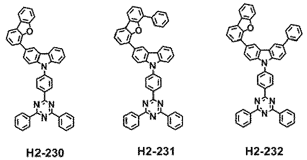

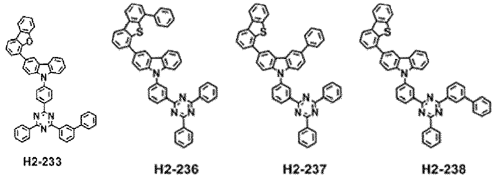

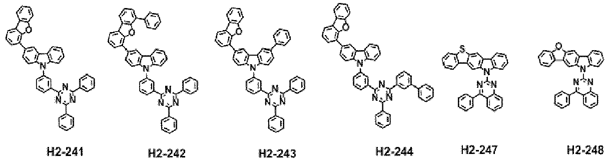

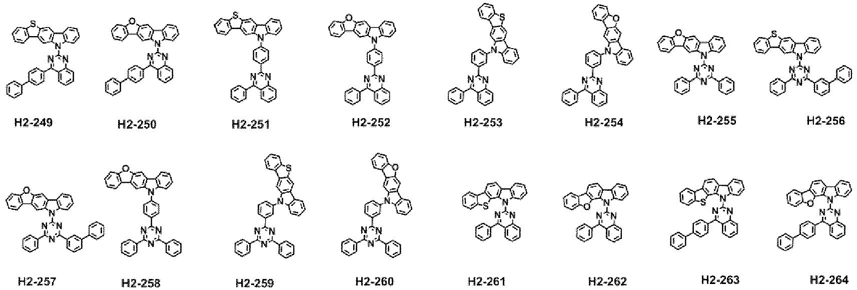

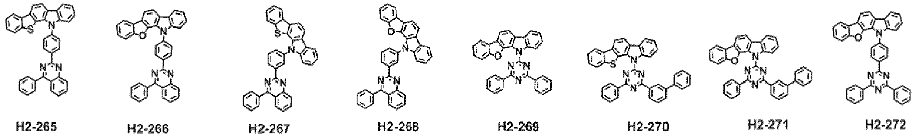

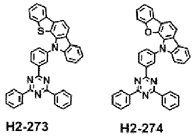

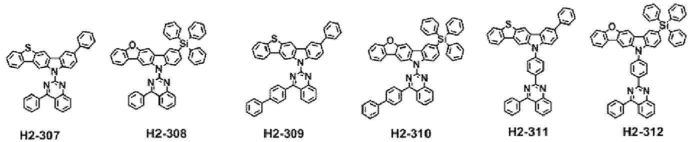

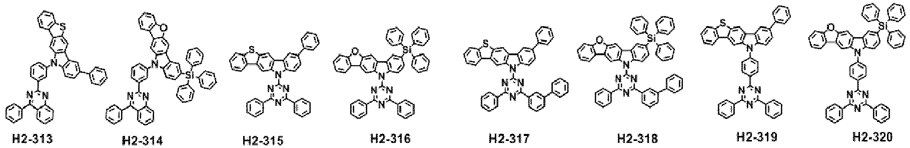

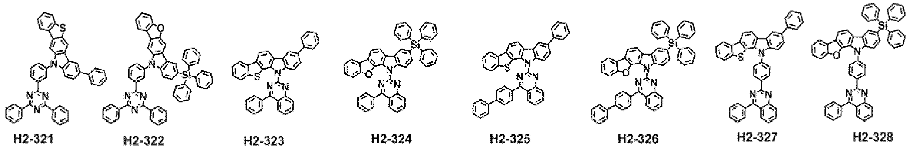

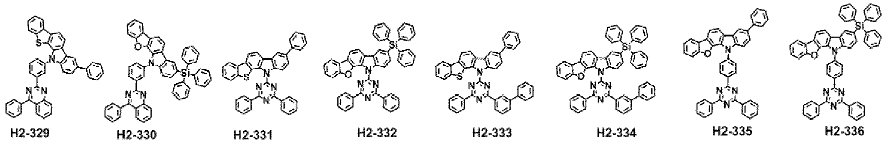

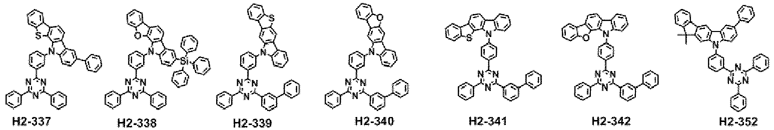

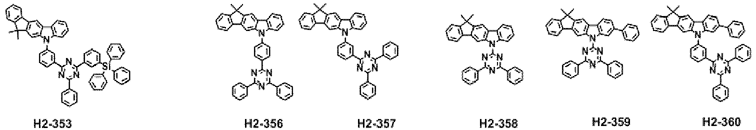

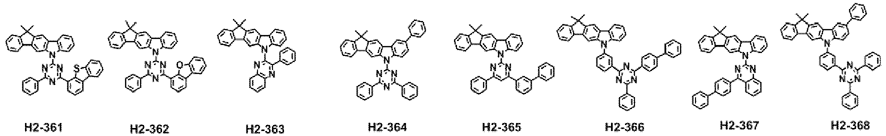

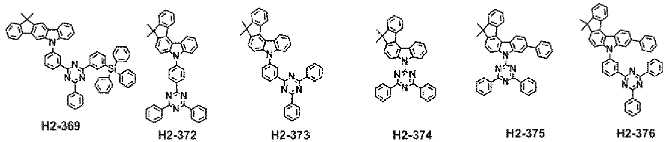

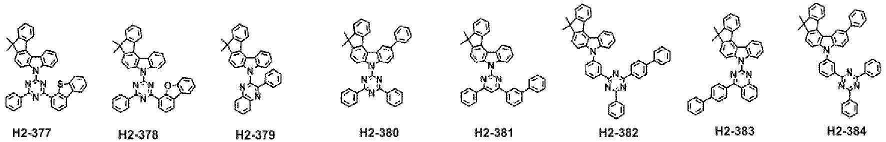

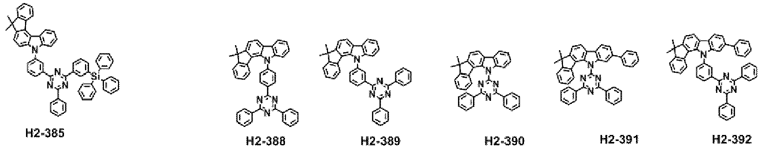

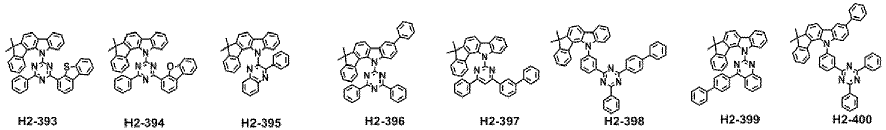

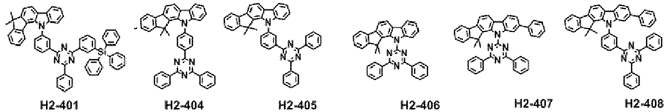

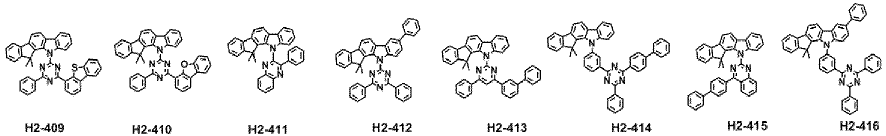

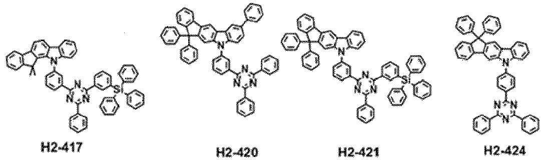

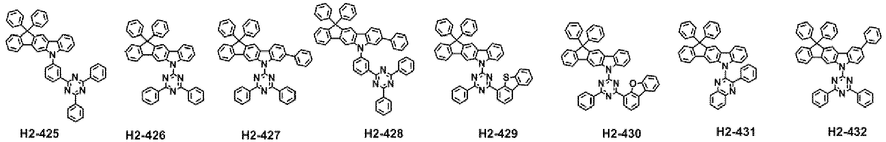

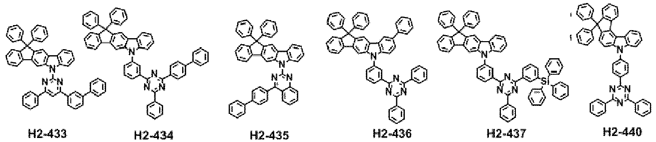

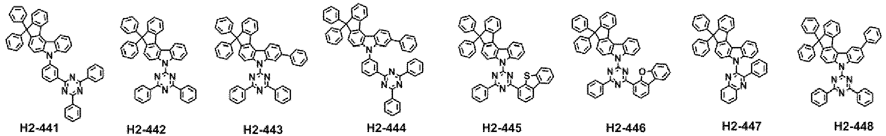

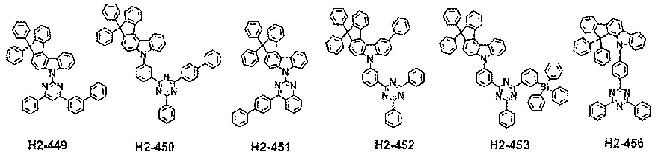

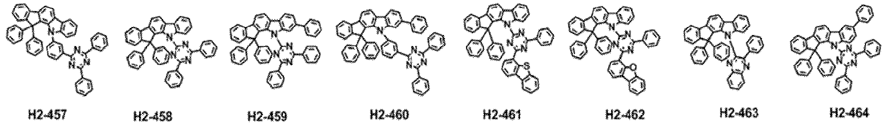

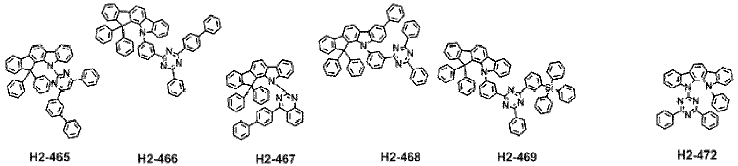

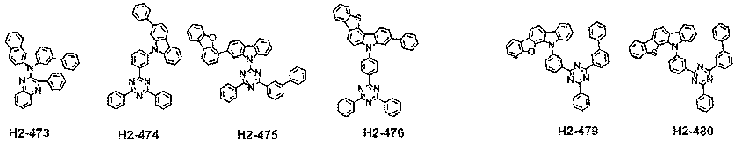

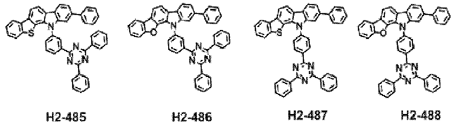

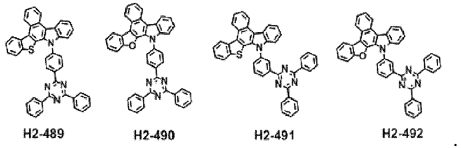

## Patentansprüche

1. Organische Elektrolumineszenzvorrichtung, umfassend mindestens eine lichtemittierende Schicht zwischen einer Anode und einer Kathode, wobei die lichtemittierende Schicht einen Wirt und einen phosphoreszierenden Dotierstoff umfasst, wobei der Wirt aus Mehrkomponentenwirtsverbindungen besteht, wobei mindestens eine erste Wirtsverbindung der Mehrkomponentenwirtsverbindungen durch die folgende Formel 1 dargestellt ist und eine zweite Wirtsverbindung durch die folgende Formel 2 dargestellt ist: 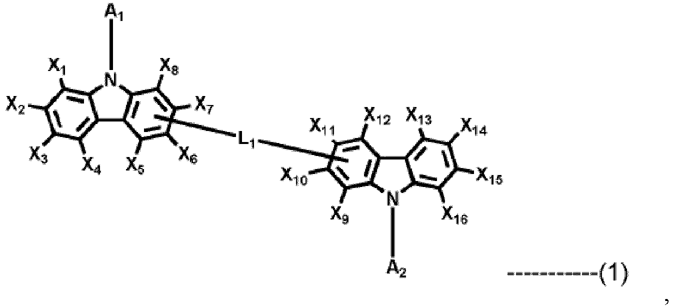 wobei
A₁ und A₂ jeweils unabhängig für ein unsubstituiertes (C6-C30)-Aryl stehen, das aus einer unsubstituierten Phenylgruppe, einer unsubstituierten Naphthylgruppe oder einer unsubstituierten Biphenylgruppe ausgewählt ist;
L₁ für ein substituiertes oder unsubstituiertes (C6-C30)-Arylen steht, das durch eine beliebige der folgenden Formeln (7) bis (11) oder (13) bis (19) dargestellt ist; 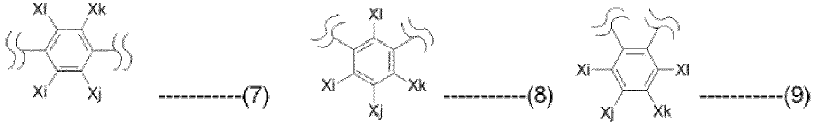 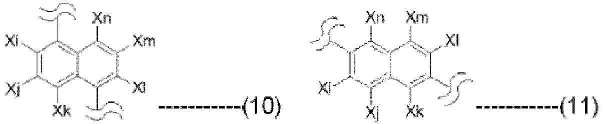 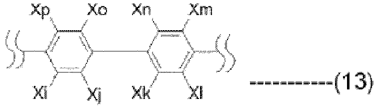 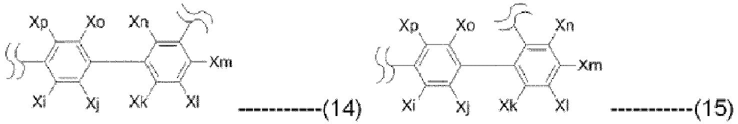 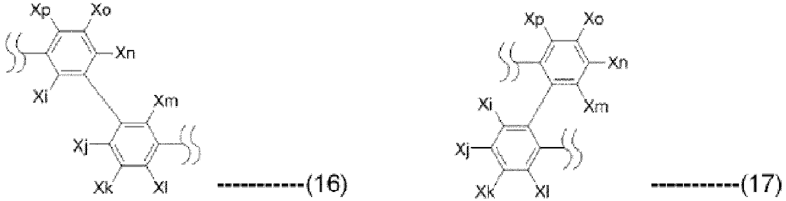 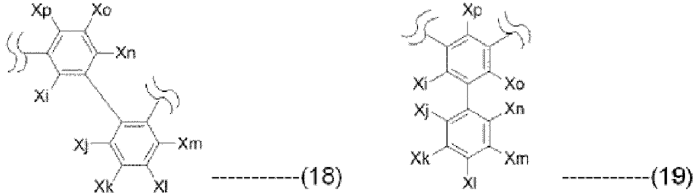
Xi bis Xp jeweils unabhängig für Wasserstoff, Deuterium, ein Halogen, ein substituiertes oder unsubstituiertes (C1-C30)-Alkyl, ein substituiertes oder unsubstituiertes (C2-C30)-Alkenyl, ein substituiertes oder unsubstituiertes (C2-C30)-Alkinyl, ein substituiertes oder unsubstituiertes (C3-C30)-Cycloalkyl, ein substituiertes oder unsubstituiertes (C6-C60)-Aryl, ein substituiertes oder unsubstituiertes (3- bis 30-gliedriges) Heteroaryl, ein substituiertes oder unsubstituiertes Tri-(C1-C30)-alkylsilyl, ein substituiertes oder unsubstituiertes Tri-(C6-C30)-arylsilyl, ein substituiertes oder unsubstituiertes Di-(C1-C30)-alkyl-(C6-C30)-arylsilyl, ein substituiertes oder unsubstituiertes (C1-C30)-Alkyldi-(C6-C30)-arylsilyl oder ein substituiertes oder unsubstituiertes Mono- oder Di-(C6-C30)-arylamino stehen oder mit einem oder mehreren benachbarten Substituenten verknüpft sind, um einen substituierten oder unsubstituierten, mono- oder polycyclischen, alicyclischen oder aromatischen (C3-C30)-Ring zu bilden, dessen Kohlenstoffatom(e) durch mindestens ein Heteroatom ersetzt sein können, das aus Stickstoff, Sauerstoff und Schwefel ausgewählt ist;
X₁ bis X₁₆ jeweils unabhängig für Wasserstoff, Deuterium, ein Halogen, ein substituiertes oder unsubstituiertes (C1-C30)-Alkyl, ein substituiertes oder unsubstituiertes (C2-C30)-Alkenyl, ein substituiertes oder unsubstituiertes (C2-C30)-Alkinyl, ein substituiertes oder unsubstituiertes (C3-C30)-Cycloalkyl, ein substituiertes oder unsubstituiertes (C6-C60)-Aryl, das aus der Gruppe bestehend aus Phenyl, Biphenyl, Terphenyl, Naphthyl, Binaphthyl, Phenylnaphthyl und Naphthylphenyl ausgewählt ist, ein substituiertes oder unsubstituiertes (3- bis 30-gliedriges) Heteroaryl, ein substituiertes oder unsubstituiertes Tri-(C1-C30)-alkylsilyl, ein substituiertes oder unsubstituiertes Tri-(C6-C30)-arylsilyl, ein substituiertes oder unsubstituiertes Di-(C1-C30)-alkyl-(C6-C30)-arylsilyl, ein substituiertes oder unsubstituiertes (C1-C30)-Alkyldi-(C6-C30)-arylsilyl oder ein substituiertes oder unsubstituiertes Mono- oder Di-(C6-C30)-arylamino stehen oder mit einem oder mehreren benachbarten Substituenten verknüpft sind, um einen substituierten oder unsubstituierten, mono- oder polycyclischen, alicyclischen oder aromatischen (C3-C30)-Ring zu bilden, dessen Kohlenstoffatom(e) durch mindestens ein Heteroatom ersetzt sein können, das aus Stickstoff, Sauerstoff und Schwefel ausgewählt ist;
die Substituenten an der substituierten Gruppe von L1, X1 bis X16 Deuterium, ein Halogen, eine unsubstituierte (C1-C30)-Alkyl-Gruppe, eine unsubstituierte (C6-C30)-Aryl-Gruppe, eine unsubstituierte (3- bis 30-gliedrige) Heteroaryl-Gruppe, ein Tri-(C6-C30)-arylsilyl, ein Tri-(C1-C30)-alkylsilyl, ein Di-(C1-C30)-alkyl-(C6-C30)-arylsilyl, ein (C1-C30)-Alkyldi-(C6-C30)-arylsilyl, ein Amino, ein Mono- oder Di-(C1-C30)-alkylamino, ein Mono- oder Di-(C6-C30)-arylamino, ein (C1-C30)-Alkyl-(C6-C30)-arylamino, ein (C6-C30)-Aryl-(C1-C30)-alkyl oder ein (C1-C30)-Alkyl-(C6-C30)-aryl sind, 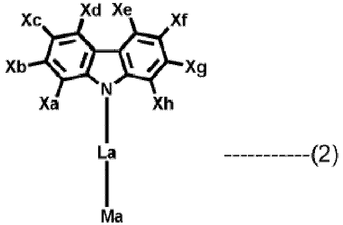 wobei
Ma für ein substituiertes oder unsubstituiertes stickstoffhaltiges (5- bis 11-gliedriges) Heteroaryl steht;
La für eine Einfachbindung oder ein substituiertes oder unsubstituiertes (C6-C30)-Arylen steht;
Xa bis Xh jeweils unabhängig für Wasserstoff, Deuterium, ein Halogen, ein substituiertes oder unsubstituiertes (C1-C30)-Alkyl, ein substituiertes oder unsubstituiertes (C2-C30)-Alkenyl, ein substituiertes oder unsubstituiertes (C2-C30)-Alkinyl, ein substituiertes oder unsubstituiertes (C3-C30)-Cycloalkyl, ein substituiertes oder unsubstituiertes (C6-C60)-Aryl, ein substituiertes oder unsubstituiertes (3- bis 30-gliedriges) Heteroaryl, ein substituiertes oder unsubstituiertes Tri-(C1-C30)-alkylsilyl, ein substituiertes oder unsubstituiertes Tri-(C6-C30)-arylsilyl, ein substituiertes oder unsubstituiertes Di-(C1-C30)-alkyl-(C6-C30)-arylsilyl oder ein substituiertes oder unsubstituiertes Mono- oder Di-(C6-C30)-arylamino stehen oder mit einem oder mehreren benachbarten Substituenten verknüpft sind, um einen substituierten oder unsubstituierten, mono- oder polycyclischen, alicyclischen oder aromatischen (C3-C30)-Ring zu bilden, dessen Kohlenstoffatom(e) durch mindestens ein Heteroatom ersetzt sein können, das aus Stickstoff, Sauerstoff und Schwefel ausgewählt ist; und
das Heteroaryl mindestens ein Heteroatom enthält, das aus B, N, O, S, P(=O), Si und P ausgewählt ist; und
die Substituenten an der substituierten Gruppe von Ma, La, Xa bis Xh Deuterium, ein Halogen, eine unsubstituierte (C1-C30)-Alkyl-Gruppe, eine unsubstituierte (C6-C30)-Aryl-Gruppe, eine unsubstituierte (3- bis 30-gliedrige) Heteroaryl-Gruppe, ein Tri-(C6-C30)-arylsilyl, ein Tri-(C1-C30)-alkylsilyl, ein Di-(C1-C30)-alkyl-(C6-C30)-arylsilyl, ein (C1-C30)-Alkyldi-(C6-C30)-arylsilyl, ein Amino, ein Mono- oder Di-(C1-C30)-alkylamino, ein Mono- oder Di-(C6-C30)-arylamino, ein (C1-C30)-Alkyl-(C6-C30)-arylamino, ein (C6-C30)-Aryl-(C1-C30)-alkyl oder ein (C1-C30)-Alkyl-(C6-C30)-aryl sind.

2. Organische Elektrolumineszenzvorrichtung nach Anspruch 1, wobei in Formel 2 Ma für ein monocyclisches Heteroaryl, das aus der Gruppe bestehend aus Pyrrolyl, Imidazolyl, Pyrazolyl, Triazinyl, Tetrazinyl, Triazolyl, Tetrazolyl, Pyridyl, Pyrazinyl, Pyrimidinyl und Pyridazinyl ausgewählt ist, oder ein anelliertes Heteroaryl, das aus der Gruppe bestehend aus Benzoimidazolyl, Isoindolyl, Indolyl, Indazolyl, Benzothiadiazolyl, Chinolyl, Isochinolyl, Cinnolinyl, Chinazolinyl, Naphthyridinyl und Chinoxalinyl ausgewählt ist, steht.

3. Organische Elektrolumineszenzvorrichtung nach Anspruch 1, wobei in Formel 2 La eine Einfachbindung ist oder durch eine der folgenden Formeln 7 bis 19 dargestellt ist: 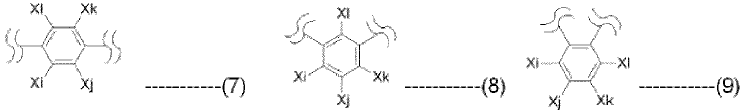 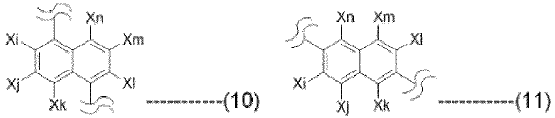 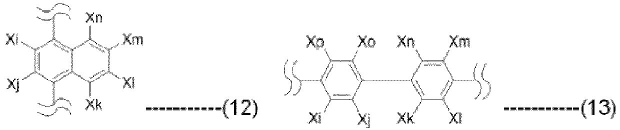 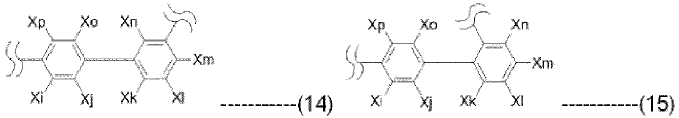 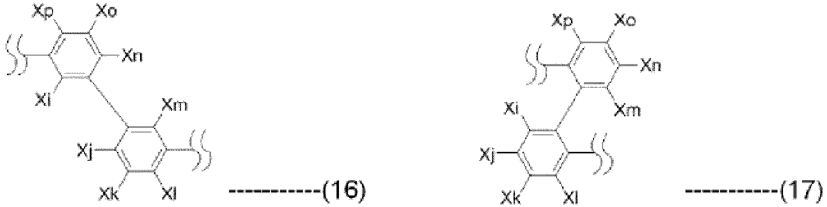 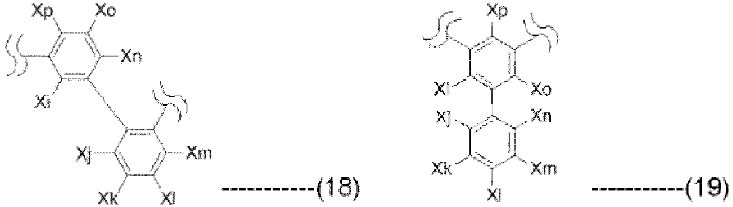 wobei
Xi bis Xp jeweils unabhängig für Wasserstoff, Deuterium, ein Halogen, ein substituiertes oder unsubstituiertes (C1-C30)-Alkyl, ein substituiertes oder unsubstituiertes (C2-C30)-Alkenyl, ein substituiertes oder unsubstituiertes (C2-C30)-Alkinyl, ein substituiertes oder unsubstituiertes (C3-C30)-Cycloalkyl, ein substituiertes oder unsubstituiertes (C6-C60)-Aryl, ein substituiertes oder unsubstituiertes (3- bis 30-gliedriges) Heteroaryl, ein substituiertes oder unsubstituiertes Tri-(C1-C30)-alkylsilyl, ein substituiertes oder unsubstituiertes Tri-(C6-C30)-arylsilyl, ein substituiertes oder unsubstituiertes Di-(C1-C30)-alkyl-(C6-C30)-arylsilyl, ein substituiertes oder unsubstituiertes (C1-C30)-Alkyldi-(C6-C30)-arylsilyl oder ein substituiertes oder unsubstituiertes Mono- oder Di-(C6-C30)-arylamino stehen oder mit einem oder mehreren benachbarten Substituenten verknüpft sind, um einen substituierten oder unsubstituierten, mono- oder polycyclischen, alicyclischen oder aromatischen (C3-C30)-Ring zu bilden, dessen Kohlenstoffatom(e) durch mindestens ein Heteroatom ersetzt sein können, das aus Stickstoff, Sauerstoff und Schwefel ausgewählt ist.

4. Organische Elektrolumineszenzvorrichtung nach Anspruch 1, wobei in Formel 2 Xa bis Xh jeweils unabhängig für Wasserstoff; ein (C6-C15)-Aryl, das unsubstituiert oder mit einem Tri-(C6-C10)-arylsilyl substituiert ist; ein (10- bis 20-gliedriges) Heteroaryl, das unsubstituiert oder mit einem (C6-C12)-Aryl oder einem Cyano-(C6-C12)-aryl substituiert ist; oder ein unsubstituiertes Tri-(C6-C10)-arylsilyl stehen oder mit einem oder mehreren benachbarten Substituenten verknüpft sind, um ein substituiertes oder unsubstituiertes Benzol, ein substituiertes oder unsubstituiertes Indol, ein substituiertes oder unsubstituiertes Benzoindol, ein substituiertes oder unsubstituiertes Inden, ein substituiertes oder unsubstituiertes Benzofuran oder ein substituiertes oder unsubstituiertes Benzothiophen zu bilden.

5. Organische Elektrolumineszenzvorrichtung nach Anspruch 1, wobei in Formel 1 ein Triarylsilyl als X₁ bis X₁₆ ein Triphenylsilyl ist.

6. Organische Elektrolumineszenzvorrichtung nach Anspruch 1, wobei die durch die Formel 1 dargestellte Verbindung ausgewählt ist aus der Gruppe bestehend aus:

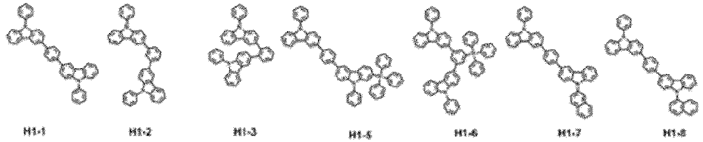

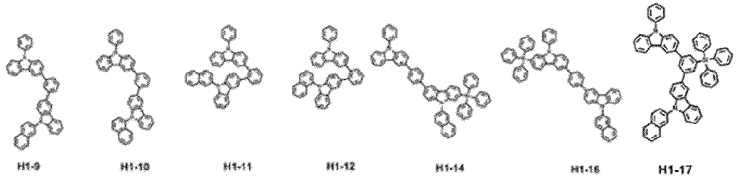

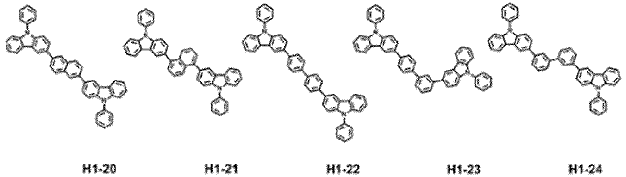

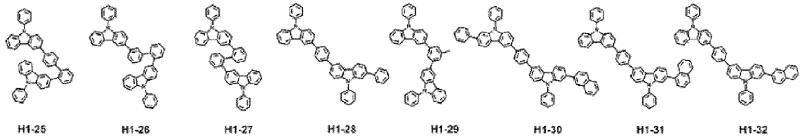

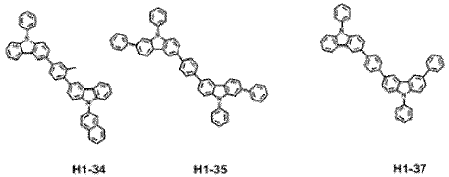

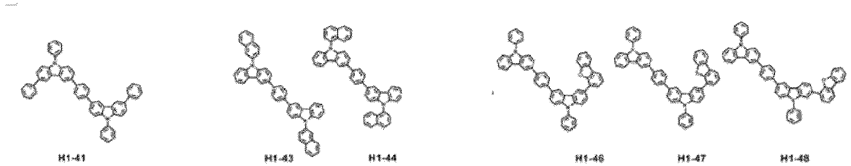

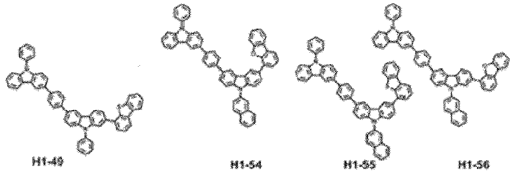

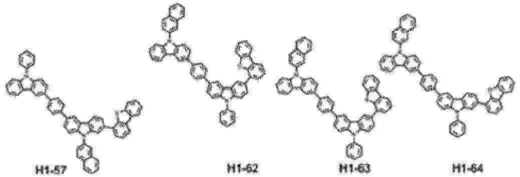

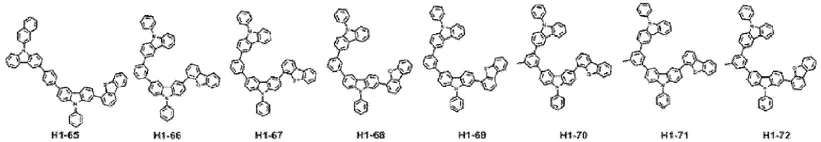

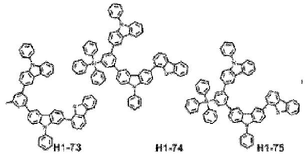

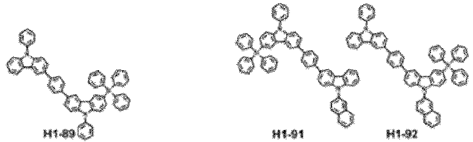

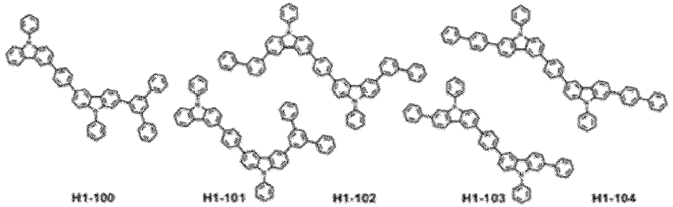

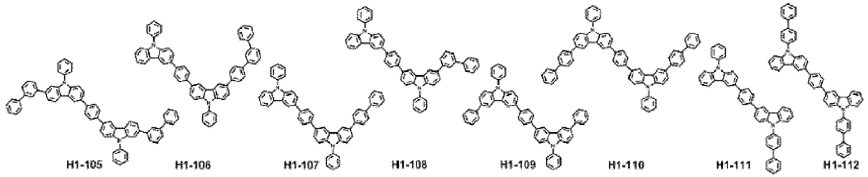

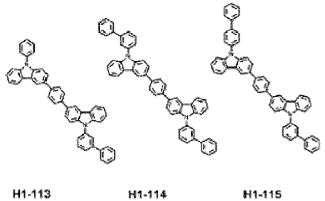

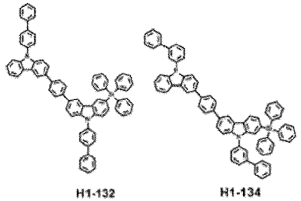

7. Organische Elektrolumineszenzvorrichtung nach Anspruch 1, wobei die durch die Formel 2 dargestellte Verbindung ausgewählt ist aus der Gruppe bestehend aus:

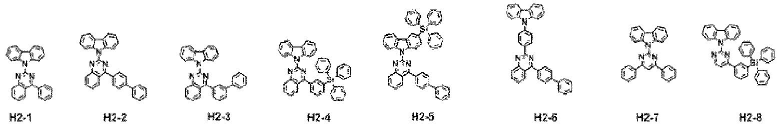

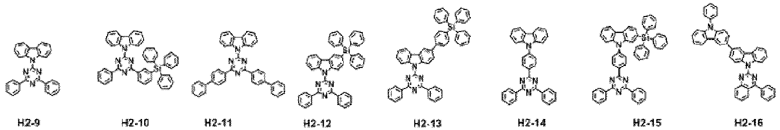

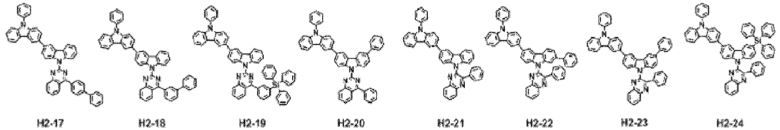

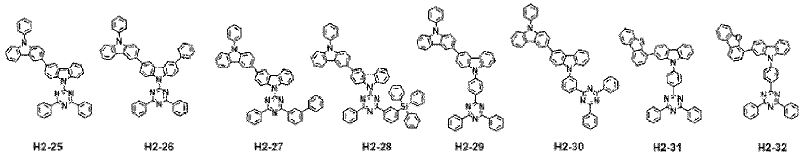

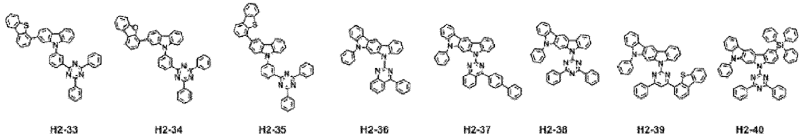

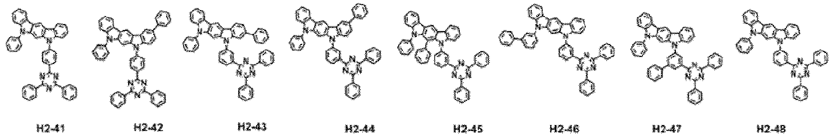

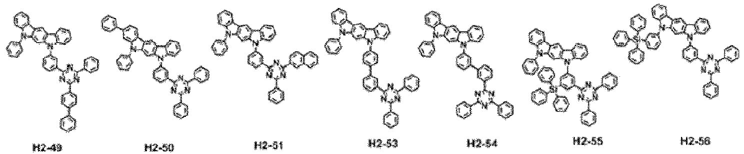

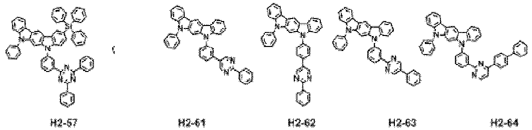

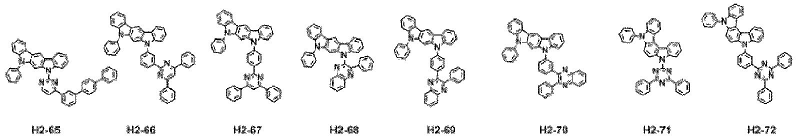

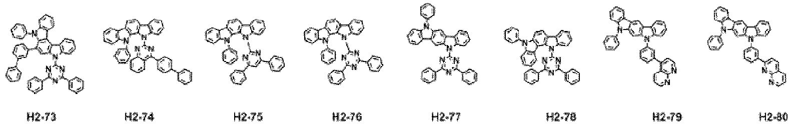

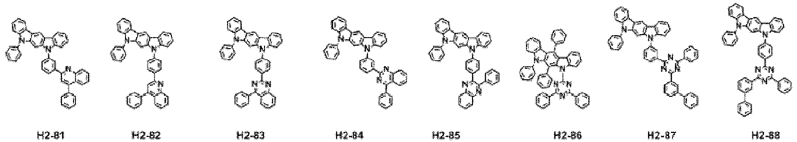

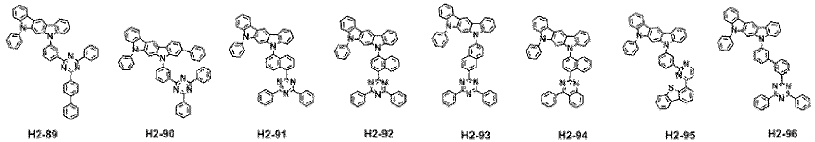

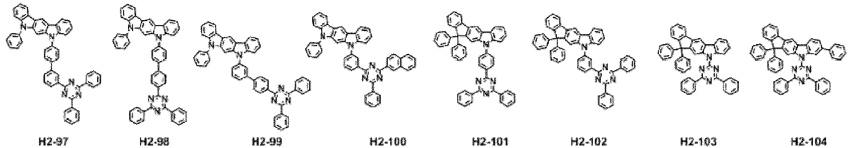

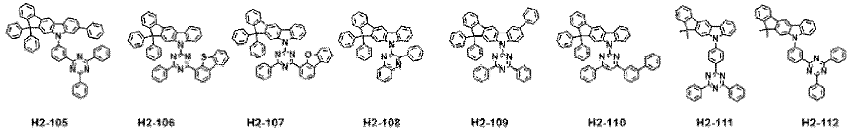

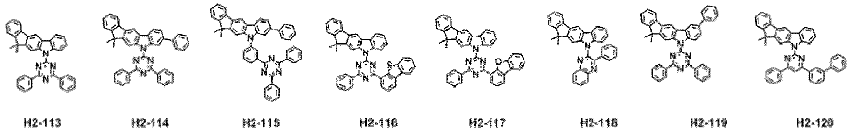

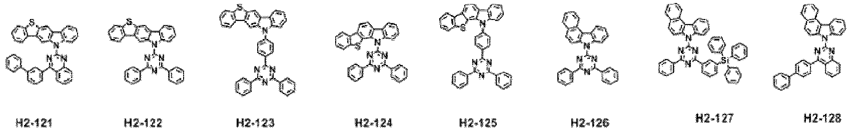

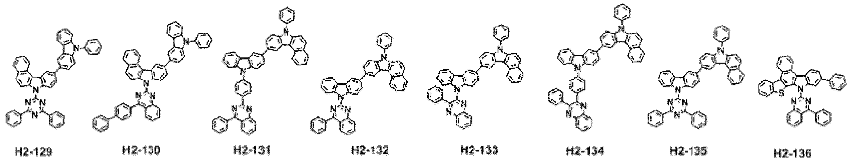

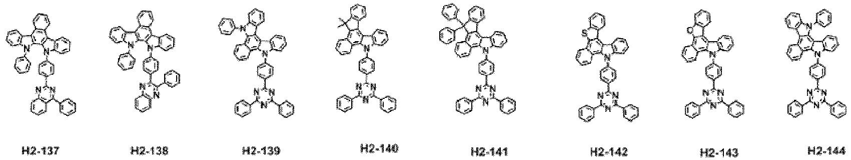

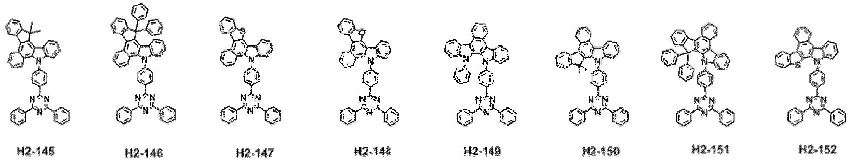

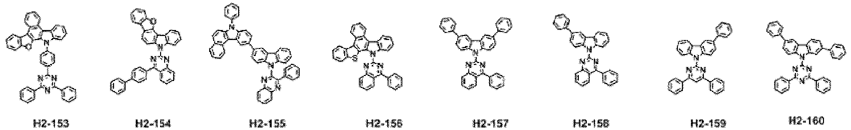

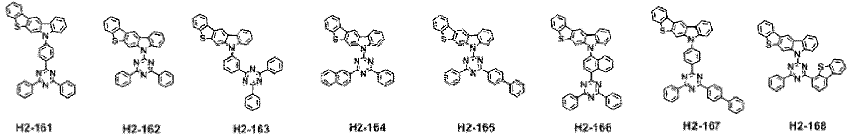

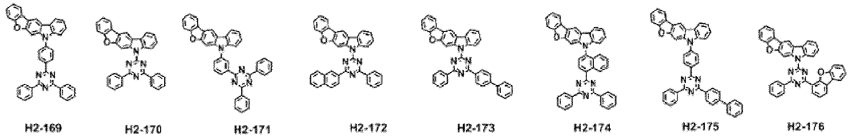

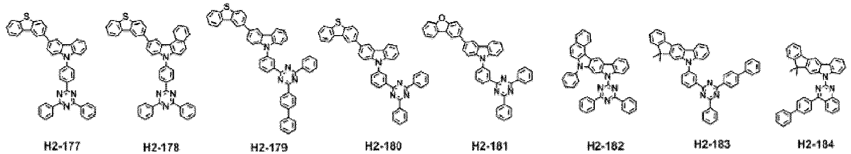

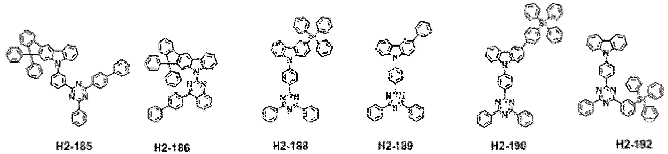

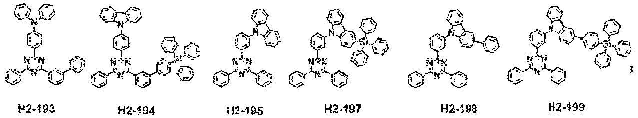

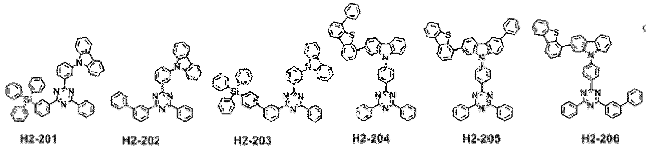

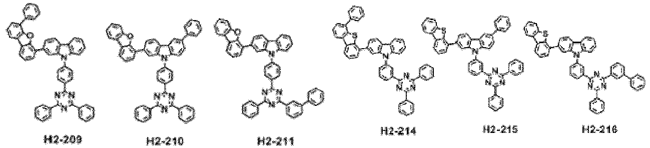

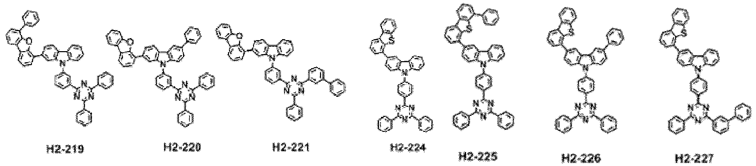

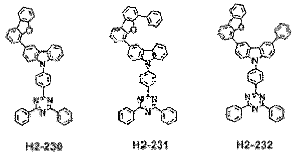

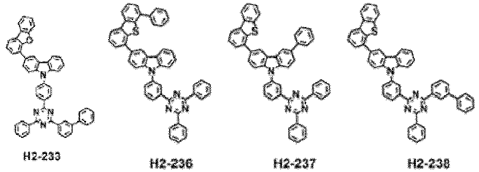

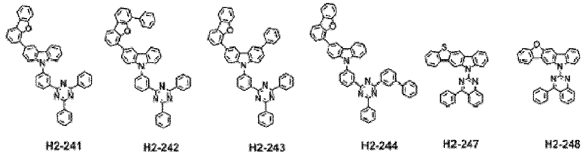

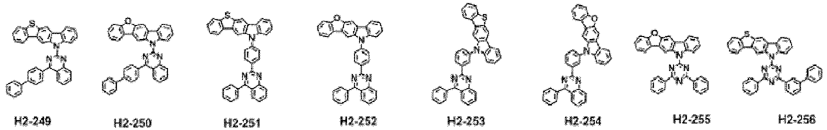

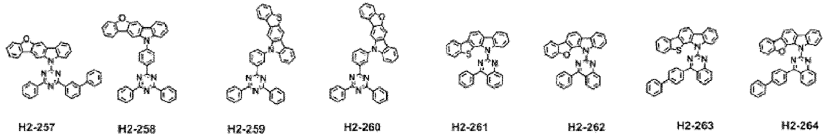

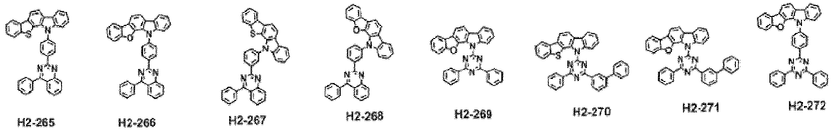

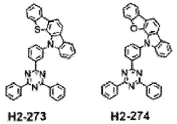

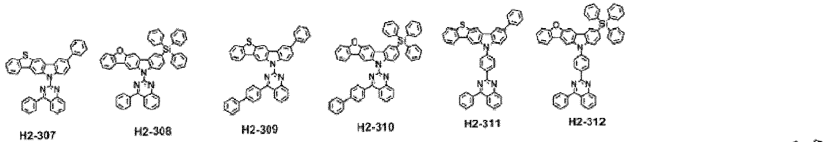

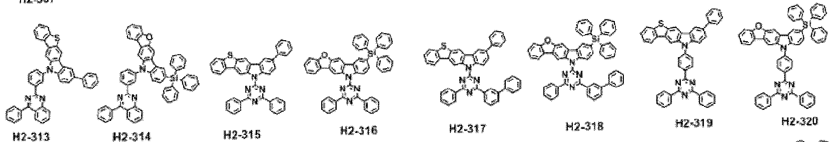

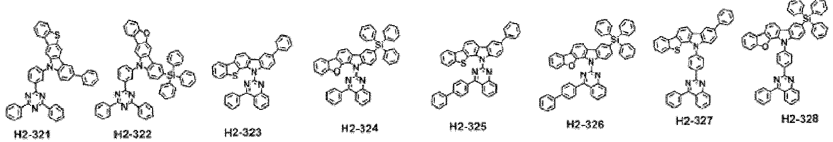

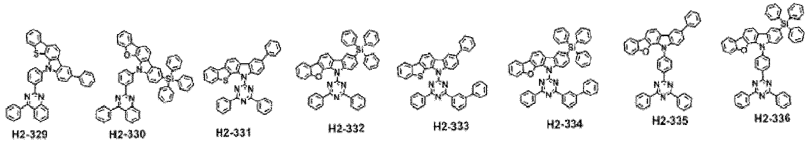

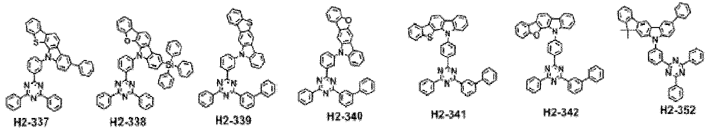

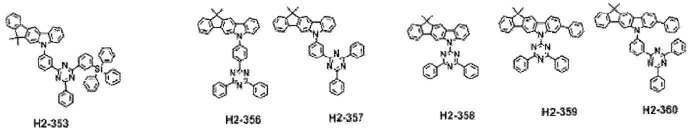

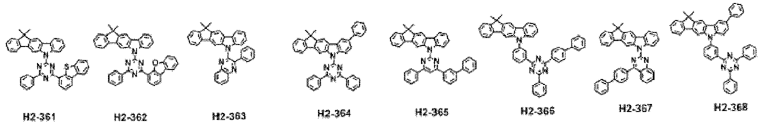

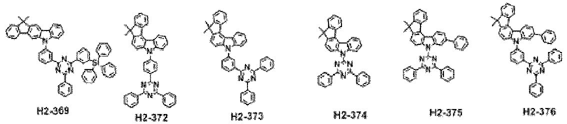

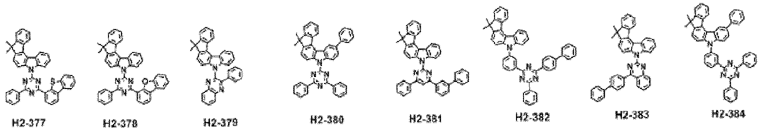

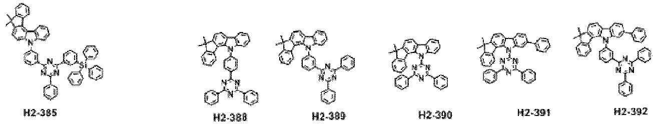

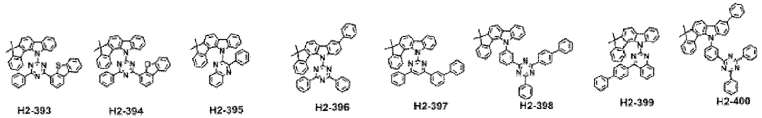

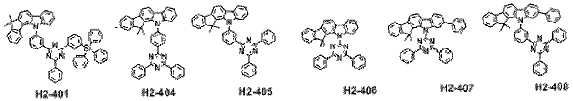

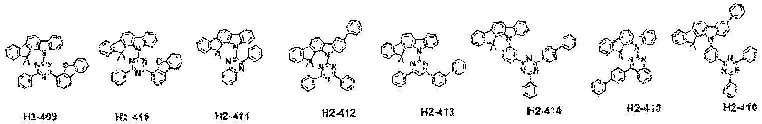

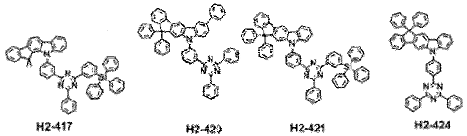

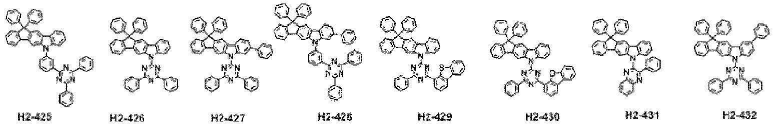

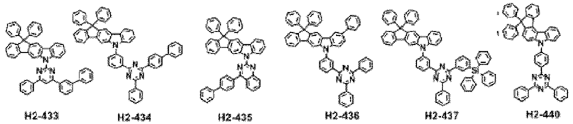

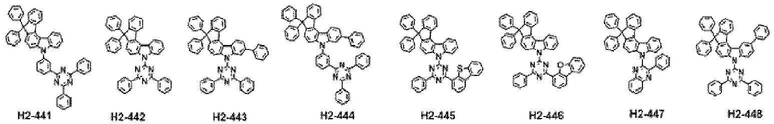

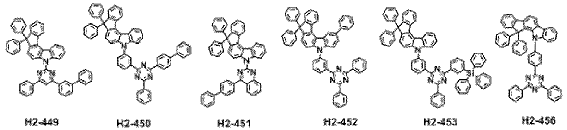

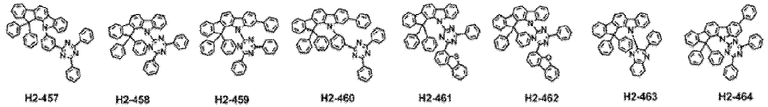

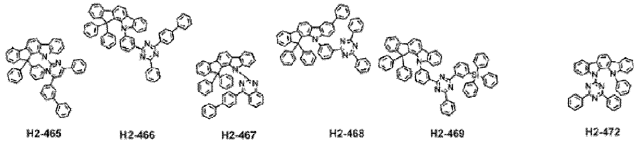

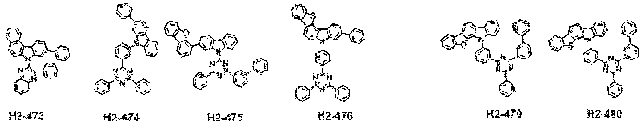

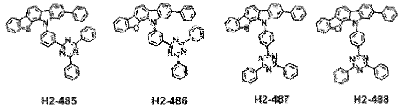

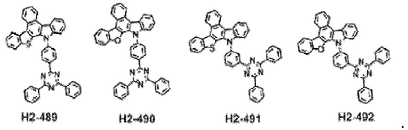

## Revendications

1. Dispositif électroluminescent organique comprenant au moins une couche électroluminescente entre une anode et une cathode, ladite couche électroluminescente comprenant un hôte et un dopant phosphorescent, ledit hôte se composant de composés hôtes multi-composants, au moins un premier composé hôte des composés hôtes multi-composants étant représenté par la formule 1 suivante, et un deuxième composé hôte étant représenté par la formule 2 suivante : 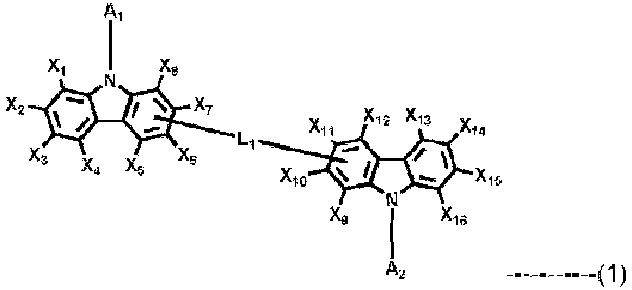 dans laquelle
A₁ et A₂ représentent chacun indépendamment un aryle en (C6-C30) non substitué choisi parmi un groupe phényle non substitué, naphtyle non substitué ou biphényle non substitué ;
L₁ représente un arylène en (C6-C30) substitué ou non substitué représenté par l'une quelconque des formules (7) à (11) ou (13) à (19) suivantes : 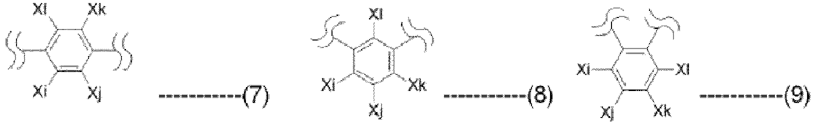 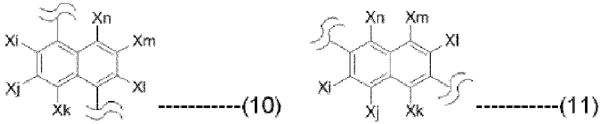 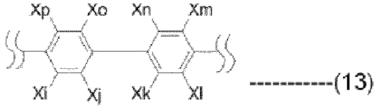 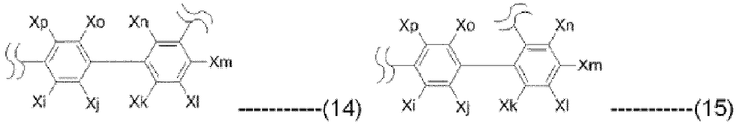 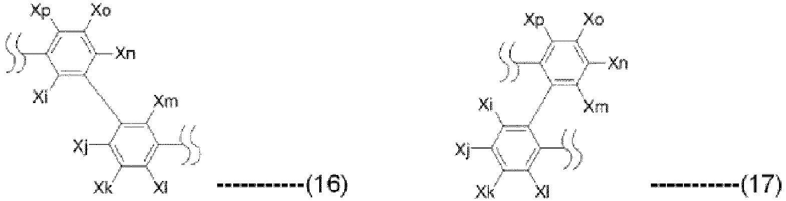 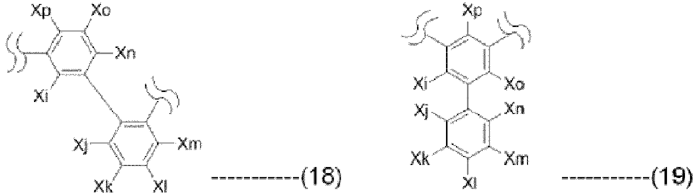
Xi à Xp représentent chacun indépendamment hydrogène, deutérium, un halogène, un alkyle en (C1-C30) substitué ou non substitué, un alcényle en (C2-C30) substitué ou non substitué, un alcynyle en (C2-C30) substitué ou non substitué, un cycloalkyle en (C3-C30) substitué ou non substitué, un aryle en (C6-C60) substitué ou non substitué, un hétéroaryle à 3 à 30 chaînons substitué ou non substitué, un tri-alkylsilyle en (C1-C30) substitué ou non substitué, un tri-arylsilyle en (C6-C30) substitué ou non substitué, un di-alkyle en (C1-C30)-arylsilyle en (C6-C30) substitué ou non substitué, un alkyle en (C1-C30)-di-arylsilyle en (C6-C30) substitué ou non substitué, ou un mono- ou di-arylamino en (C6-C30) substitué ou non substitué ; ou sont liés à un ou plusieurs substituants adjacents pour former un cycle alicyclique ou aromatique, mono- ou polycyclique, en (C3-C30) substitué ou non substitué dont le ou les atomes de carbone peuvent être remplacés par au moins un hétéroatome choisi parmi l'azote, l'oxygène et le soufre ;
X₁ à X₁₆ représentent chacun indépendamment hydrogène, deutérium, un halogène, un alkyle en (C1-C30) substitué ou non substitué, un alcényle en (C2-C30) substitué ou non substitué, un alcynyle en (C2-C30) substitué ou non substitué, un cycloalkyle en (C3-C30) substitué ou non substitué, un aryle en (C6-C60) substitué ou non substitué choisi dans le groupe constitué par phényle, biphényle, terphényle, naphtyle, binaphtyle, phénylnaphtyle et naphtylphényle, un hétéroaryle à 3 à 30 chaînons substitué ou non substitué, un tri-alkylsilyle en (C1-C30) substitué ou non substitué, un tri-arylsilyle en (C6-C30) substitué ou non substitué, un di-alkyle en (C1-C30)-arylsilyle en (C6-C30) substitué ou non substitué, un alkyle en (C1-C30)-di-arylsilyle en (C6-C30) substitué ou non substitué, ou un mono- ou di-arylamino en (C6-C30) substitué ou non substitué ; ou sont liés à un ou plusieurs substituants adjacents pour former un cycle alicyclique ou aromatique, mono- ou polycyclique, en (C3-C30) substitué ou non substitué dont le ou les atomes de carbone peuvent être remplacés par au moins un hétéroatome choisi parmi l'azote, l'oxygène et le soufre ;
les substituants présents sur le groupe substitué de L1, X1 à X16 sont : deutérium, un halogène, un groupe alkyle en (C1-C30) non substitué, un groupe aryle en (C6-C30) non_substitué, un groupe hétéroaryle à 3 à 30 chaînons non substitué, un tri-arylsilyle en (C6-C30), un tri-alkylsilyle en (C1-C30), un di-alkyle en (C1-C30)-arylsilyle en (C6-C30), un alkyle en (C1-C30)-di-arylsilyle en (C6-C30), un amino, un mono- ou di-alkylamino en (C1-C30), un mono- ou di-arylamino en (C6-C30), un alkyle en (C1-C30)-arylamino en (C6-C30), un aryle en (C6-C30)-alkyle en (C1-C30) ou un alkyle en (C1-C30)-aryle en (C6-C30) ; 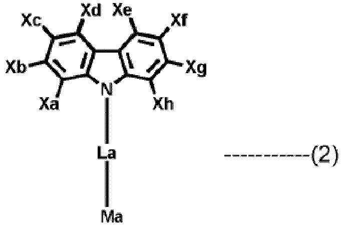 dans laquelle
Ma représente un hétéroaryle à 5 à 11 chaînons, contenant de l'azote, substitué ou non substitué ;
La représente une liaison simple, ou un arylène en (C6-C30) substitué ou non substitué ;
Xa à Xh représentent chacun indépendamment hydrogène, deutérium, un halogène, un alkyle en (C1-C30) substitué ou non substitué, un alcényle en (C2-C30) substitué ou non substitué, un alcynyle en (C2-C30) substitué ou non substitué, un cycloalkyle en (C3-C30) substitué ou non substitué, un aryle en (C6-C60) substitué ou non substitué, un hétéroaryle à 3 à 30 chaînons substitué ou non substitué, un tri-alkylsilyle en (C1-C30) substitué ou non substitué, un tri-arylsilyle en (C6-C30) substitué ou non substitué, un di-alkyle en (C1-C30)-arylsilyle en (C6-C30) substitué ou non substitué, ou un mono- ou di-arylamino en (C6-C30) substitué ou non substitué ; ou sont liés à un ou plusieurs substituants adjacents pour former un cycle alicyclique ou aromatique, mono- ou polycyclique, en (C3-C30) substitué ou non substitué dont le ou les atomes de carbone peuvent être remplacés par au moins un hétéroatome choisi parmi l'azote, l'oxygène et le soufre ; et
l'hétéroaryle contient au moins un hétéroatome choisi parmi B, N, O, S, P(=O), Si et P ; et
les substituants présents sur le groupe substitué de Ma, La, Xa à Xh sont deutérium, un halogène, un groupe alkyle en (C1-C30) non substitué, un groupe aryle en (C6-C30) non substitué, un groupe hétéroaryle à 3 à 30 chaînons non substitué, un tri-arylsilyle en (C6-C30), un tri-alkylsilyle en (C1-C30), un di-alkyle en (C1-C30)-arylsilyle en (C6-C30), un alkyle en (C1-C30)-di-arylsilyle en (C6-C30), un amino, un mono- ou di-alkylamino en (C1-C30), un mono- ou di-arylamino en (C6-C30), un alkyle en (C1-C30)-arylamino en (C6-C30), un aryle en (C6-C30)-alkyle en (C1-C30) ou un alkyle en (C1-C30)-aryle en (C6-C30).

2. Dispositif électroluminescent organique selon la revendication 1, dans lequel, dans la formule 2, Ma représente un hétéroaryle monocyclique choisi dans le groupe constitué par pyrrolyle, imidazolyle, pyrazolyle, triazinyle, tétrazinyle, triazolyle, tétrazolyle, pyridyle, pyrazinyle, pyrimidinyle et pyridazinyle, ou un hétéroaryle fusionné choisi dans le groupe constitué par benzoimidazolyle, isoindolyle, indolyle, indazolyle, benzothiadiazolyle, quinolyle, isoquinolyle, cinnolinyle, quinazolinyle, naphtyridinyle et quinoxalinyle.

3. Dispositif électroluminescent organique selon la revendication 1, dans lequel, dans la formule 2, La est une liaison simple, ou est représenté par l'une des formules 7 à 19 suivantes : 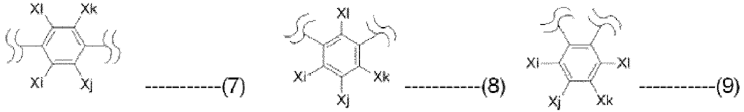 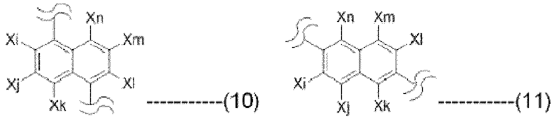 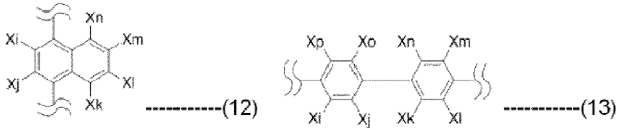 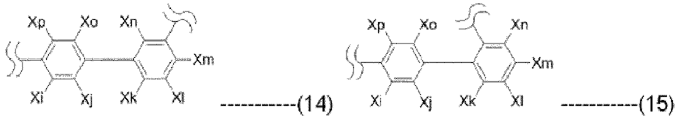 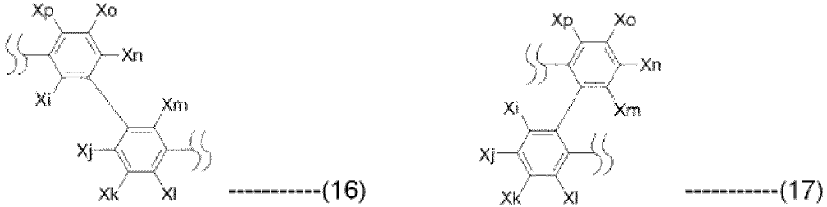 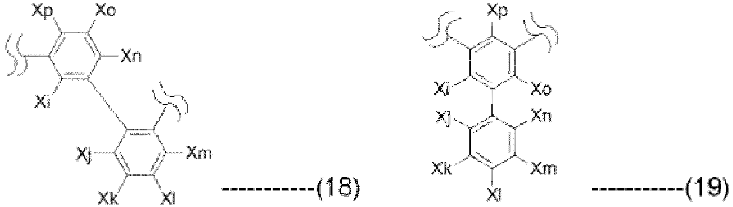 dans lesquelles
Xi à Xp représentent chacun indépendamment hydrogène, deutérium, un halogène, un alkyle en (C1-C30) substitué ou non substitué, un alcényle en (C2-C30) substitué ou non substitué, un alcynyle en (C2-C30) substitué ou non substitué, un cycloalkyle en (C3-C30) substitué ou non substitué, un aryle en (C6-C60) substitué ou non substitué, un hétéroaryle à 3 à 30 chaînons substitué ou non substitué, un tri-alkylsilyle en (C1-C30) substitué ou non substitué, un tri-arylsilyle en (C6-C30) substitué ou non substitué, un di-alkyle en (C1-C30)-arylsilyle en (C6-C30) substitué ou non substitué, un alkyle en (C1-C30)-di-arylsilyle en (C6-C30) substitué ou non substitué, ou un mono- ou di-arylamino en (C6-C30) substitué ou non substitué ; ou sont liés à un ou plusieurs substituants adjacents pour former un cycle alicyclique ou aromatique, mono- ou polycyclique, en (C3-C30) substitué ou non substitué dont le ou les atomes de carbone peuvent être remplacés par au moins un hétéroatome choisi parmi l'azote, l'oxygène et le soufre.

4. Dispositif électroluminescent organique selon la revendication 1, dans lequel, dans la formule 2, Xa à Xh représentent chacun indépendamment hydrogène ; un aryle en (C6-C15) non substitué ou substitué par un tri-arylsilyle en (C6-C10) ; un hétéroaryle à 10 à 20 chaînons non substitué ou substitué par un aryle en (C6-C12) ou un cyano-aryle en (C6-C12) ; ou un tri-arylsilyle en (C6-C10) non substitué ; ou sont liés à un ou plusieurs substituants adjacents pour former un benzène substitué ou non substitué, un indole substitué ou non substitué, un benzoindole substitué ou non substitué, un indène substitué ou non substitué, un benzofurane substitué ou non substitué ou un benzothiophène substitué ou non substitué.

5. Dispositif électroluminescent organique selon la revendication 1, dans lequel, dans la formule 1, un triarylsilyle en tant que X₁ à X₁₆ est un triphénylsilyle.

6. Dispositif électroluminescent organique selon la revendication 1, dans lequel le composé représenté par la formule 1 est choisi dans le groupe constitué par :

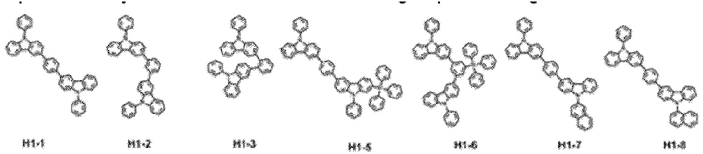

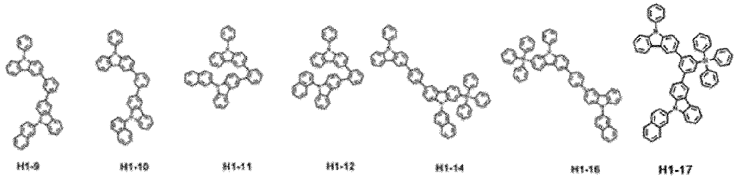

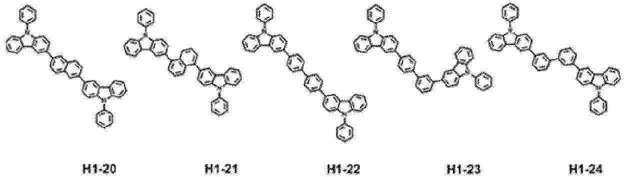

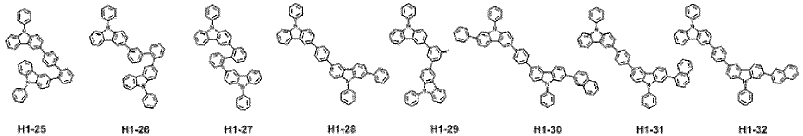

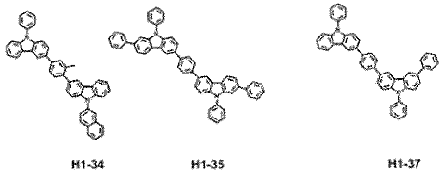

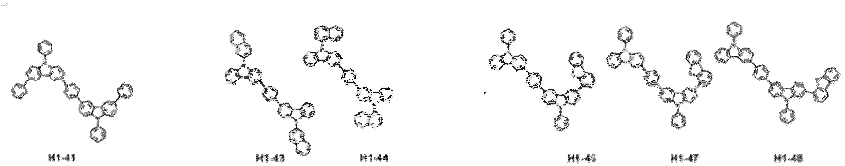

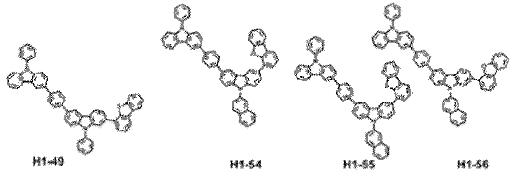

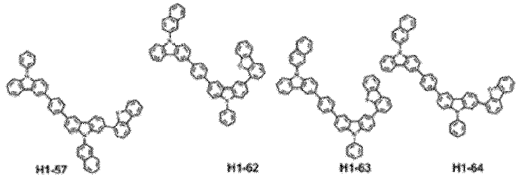

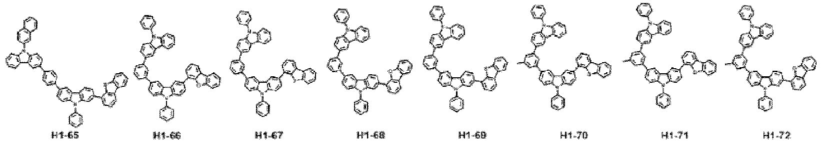

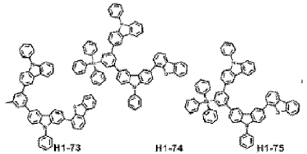

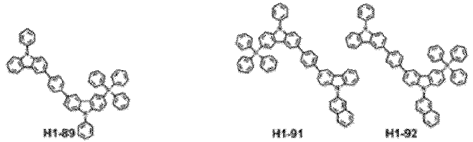

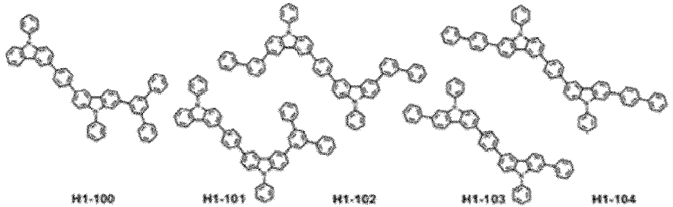

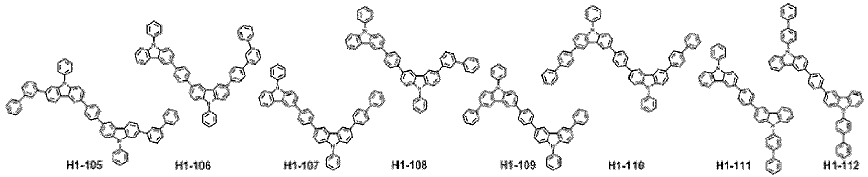

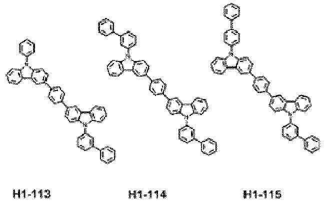

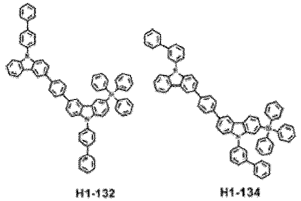

7. Dispositif électroluminescent organique selon la revendication 1, dans lequel le composé représenté par la formule 2 est choisi dans le groupe constitué par :
